(19) Europäisches Patentamt
European Patent Office
Office européen des brevets

(11) **EP 2 059 163 B1**

(12) **EUROPÄISCHE PATENTSCHRIFT**

(45) Veröffentlichungstag und Bekanntmachung des Hinweises auf die Patenterteilung:
**17.11.2010 Patentblatt 2010/46**

(51) Int Cl.:
*A61B 5/00* (2006.01)    *A61B 5/0456* (2006.01)
*G06F 17/00* (2006.01)    *G06F 19/00* (2006.01)

(21) Anmeldenummer: **07803212.5**

(22) Anmeldetag: **04.09.2007**

(86) Internationale Anmeldenummer:
**PCT/EP2007/059248**

(87) Internationale Veröffentlichungsnummer:
**WO 2008/028912 (13.03.2008 Gazette 2008/11)**

(54) **VERFAHREN ZUR ABLEITUNG UND AUSWERTUNG VON HERZ-KREISLAUF-INFORMATIONEN AUS HERZSTROMKURVEN, INSBESONDERE FÜR TELEMEDIZINISCHE ANWENDUNGEN**

METHOD FOR DERIVING AND EVALUATING CARDIOVASCULAR INFORMATION FROM CURVES OF THE CARDIAC CURRENT, IN PARTICULAR FOR APPLICATIONS IN TELEMEDICINE

PROCÉDÉ DE DÉDUCTION ET D'ÉVALUATION D'INFORMATIONS CARDIOVASCULAIRES À PARTIR DE COURBES DU COURANT CARDIAQUE, EN PARTICULIER POUR DES UTILISATIONS TÉLÉMÉDICALES

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **07.09.2006 AT 14992006**

(43) Veröffentlichungstag der Anmeldung:
**20.05.2009 Patentblatt 2009/21**

(73) Patentinhaber: **TELOZO GMBH**
**1300 Wien-Flughafen (AT)**

(72) Erfinder:
• **KRAUSS, Manfred**
  **09127 Chemnitz (DE)**
• **SCHLUND, Joachim**
  **09126 Chemnitz (DE)**
• **HARTMANN, Olaf**
  **07747 Jena (DE)**
• **SCHUBERT, Jörg**
  **09125 Chemnitz (DE)**

(74) Vertreter: **Meissner, Bolte & Partner Anwaltssozietät GbR Widenmayerstrasse 48 80538 München (DE)**

(56) Entgegenhaltungen:
**EP-A- 0 256 886        EP-A- 1 157 659
EP-A- 1 459 681        EP-A- 1 611 843
WO-A-98/09226        DE-A1-102006 002 045
US-A- 4 993 423        US-A1- 2004 193 064
US-A1- 2006 020 218        US-A1- 2006 122 470**

• **"Heart rate variability. Standards of measurement, physiological interpretation, and clinical use. Task Force of the European Society of Cardiology and the North American Society of Pacing and Electrophysiology" EUROPEAN HEART JOURNAL, THE EUROPEAN SOCIETY OF CARDIOLOGY, XX, Bd. 17, Nr. 3, März 1996 (1996-03), Seiten 354-381, XP002342325 ISSN: 0195-668X in der Anmeldung erwähnt**
• **SCHWAB J. O. ET AL.: "Nichtinvasive Risikostratifikation bei koronarer Herzerkrankung: Stellenwert des Langzeit-EKG" DEUTSCHES ÄRZTEBLATT, Bd. 44, 31. Oktober 2003 (2003-10-31), Seiten A2878-A2882, XP002468386**

**Beschreibung**

Die Erfindung betrifft ein Verfahren zur Ableitung und Auswertung von Herz-Kreislauf-Informationen aus Herzstromkurven insbesondere für telemedizinische Anwendungen.

Auf Grund der Progression von Herz-Kreislauf-Erkrankungen und der eingeschränkten therapeutischen Möglichkeiten in den Spätstadien besteht die Forderung nach einer frühzeitigen und komplexen Herz-Kreislauf-Diagnose, wobei hierbei vor allem auch das Zusammenwirken des Herzens mit dem übrigen peripheren Kreislaufsystem zum gleichen Zeitpunkt sowie zu jeder Tageszeit bewertet werden muss. Die meisten bisher bekannten Messverfahren in ihrer bisherigen Auswertung weisen Nachteile sowohl für den Patienten, als auch für die abzuleitende Diagnose durch den behandelnden Arzt auf. Telemetrische Erfordernisse finden kaum Beachtung.

Kardiovaskulär stellt die Herzstromkurve "Elektrokardiogramm (EKG)" mit den bekannten Extremitäten- und Thorax-Ableitungen die am weitesten verbreitete Diagnostik- und Überwachungsmethode dar, auch wenn sie mit einer Reihe von Unzulänglichkeiten verbunden ist, da das EKG elektrische Phänomene am Herzen erfasst und somit nicht ausreichend das gesamte Herzkreislaufsystem abbildet. In diesem Sinne muss die Forderung zur Bestimmung von möglichst umfassend im EKG enthaltenen weiteren Informationen gestellt werden. Unter Berücksichtigung einer telemetrischen Anwendung gelten folgende Schwerpunkte an eine zu schaffende Vorrichtung:

- einfaches und sicheres Handling,
- ungefährlich für Benutzer,
- kostengünstig,
- einfache und modulare Auswertungsmöglichkeit sowohl für den Arzt als auch Anwender.

Telemedizin steht allgemein als weit gefasster Sammelbegriff für die Anwendung multimedialer Kommunikations- und Informationstechnologien im Gesundheitswesen. Im engeren Sinne wird damit der konkrete Einsatz unterschiedlicher Techniken zur Erbringung individueller medizintechnischer Dienst- oder Service-Leistungen bei gleichzeitiger Überbrückung der räumlichen Trennung zwischen Arzt und Patient beschrieben.

Somit erscheint es mittels Telemedizin möglich, das relative Risiko für eine häufig zu späte Inanspruchnahme medizinischer Hilfe und damit eine Verschlechterung der Prognose zu vermindern. Wesentliche Voraussetzung hierfür sind die Praktikabilität und Zuverlässigkeit der vom Nutzer selbst abgeleiteten und telemetrisch übertragenen Herz-Kreislauf-Parameter.

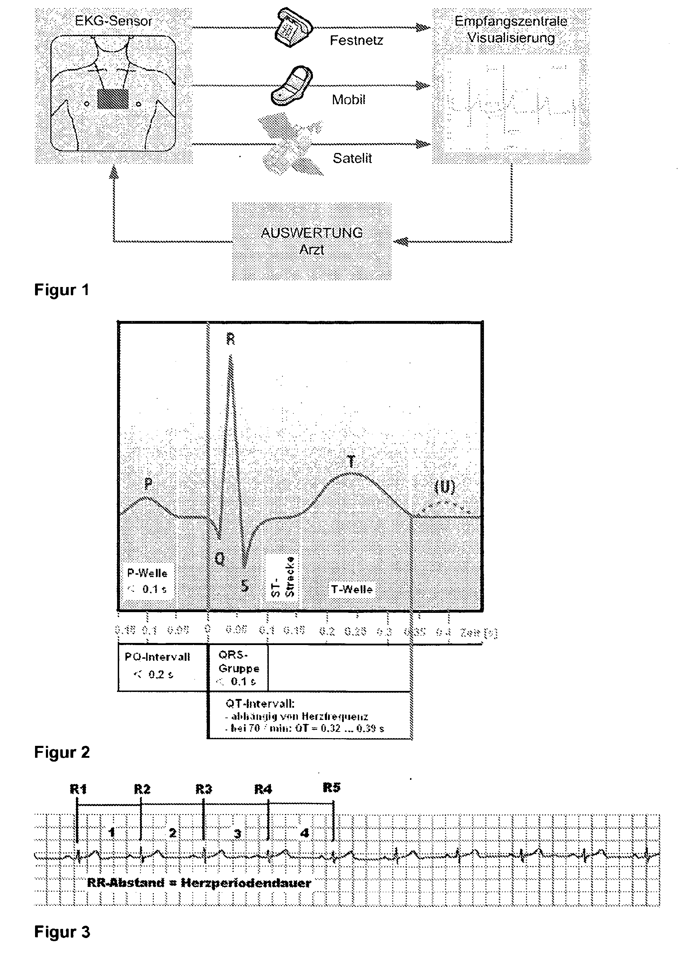

Figur 1 zeigt das allgemeine Funktionsschema bei einem Tele-EKG: aufzeichnen, senden, empfangen, auswerten. Bedeutsam hierfür erscheint das Ergebnis durchgeführter Analysen: Patienten, die dem telemedizinischen Service angeschlossen waren, reagieren schnell und zielstrebig. Nahezu 90 % nehmen innerhalb einer Stunde nach Beschwerdebeginn Kontakt zum telemedizinischen Zentrum auf.

Bei Anwendung von telemedizinischen Verfahren stellen sich besondere Anforderungen, die bei der bekannten EKG-Ableitung kaum vorliegen.

Auf dem Gebiet der mobilen Erfassung und Speicherung von Elektrokardiogramm-Signalen für Anwendungen speziell im Medizin- und Sportbereich besteht ein breiter Stand der Technik. Angeführt seien z.B. DE 197 49 768 A1 (Pflaster mit Datenaufzeichnungsfunktion zur Erfassung und Speicherung von Elektrokardiogramm-Signalen), PCT/DE98/03225 (Auswerten von Elektrokardiogrammen im Bereich von Extrasystolen), PCT/US99/09336 (Electrocardiography Electrodes Holder), DE 198 30 316 A1 (Verfahren und Vorrichtung zum Erfassen eines Kammerflimmerns), WO 99/04687 (Remote Monitoring Apparatus for Medical Conditions), DE 199 02 253 A1 (Verfahren und System zur Charakterisierung der Qualität von Herzfunktionsanzeigesignalen), PCT/US99/03666 (A System and Method for Detecting and Locating Heart Disease), PCT/US98/16693 (Automatic Cardiometer), EP 0 760 225 A1 (Method and apparatus for correcting for non-physiological variations in ECG signals), EP 0 800 787 A1 (Device for monitoring measurements electrodes, devised for picking up physiological measurement signals, and their leads), EP 0 855 167 A1 (Multiple ECG electrode strip), EP 0 864 294 A2 (Detecting abnormal activation of the heart), PCT/GB98/00742 (Method of Analysing a cardiac signal), DE 195 23 199 A1 (Verfahren zur Darstellung von EKG-Signalen), DE 195 10 382 A1 (Tragbare Vorrichtung zur Erfassung von EKG-Daten), PCT/US96/02826 (Method and apparatus for detecting artifacts using commonmode signals in differential signal detectors), PCT/BE95/00123 (A device and a method for recording and monitoring cardiac activity signals), EP 0 657 136 A1 (Electrocardiograph), DE 199 54 856 A1 (Verfahren und Vorrichtung zum Feststellen eines Anschlußfehlers bei einem telemetrischen biomedizinschen Gerät), DE 200 08 602 U1 (Volks-EKG), PCT/AU00/00656 (Cycling event and auto-trigger memory handling), PCT/US99/18296 (Ambulatory physio-kinetic monitor), DE/EP 0 746 229 T1 (Messung und Auswertung der elektrischen Stabilität des Herzens), PCT/IL00/00506 (Compact electrode assembly for a portable ECG signaling device), EP 1 110 502 A2 (Clinical research workstation), DE 100 65 578 A1 (Verfahren zur Identifizierung einer Elektroderiplatzierung), DE 102 47 435 A1 (Interpretations-Handelektrokardiograph), EP 1 157 659 A1 (Method and apparatus for reducing noise and detecting electrode faults in medical equipment), DE 102 45 143 A1 (Auswerteverfahren für Elektrokardiogramme), DE 102 33 071 A1 (Verfahren sowie System zur Messung, zur Analyse und zur drahtlosen Übertragung von Herzpotentialen), WO 02/24068 A1 (Method and system for detection of cardiac arrhythmia), DE 201 18 850 U1 (Tragbares Messgerät zur EKG-Analyse), DE 203 09 617 U1 (Simulationsvorrichtung zur Abgabe von Testsignalen an Elektrokardiographen, Gebrauchsmusterschrift), DE 203 06 009 U1 (System zur Messung, zur Analyse und zur drahtlosen Übertragung von Herzpotentialen, Gebrauchsmusterschrift), DE 299 24 092 U1 (EKG-Recorder, Gebrauchsmusterschrift), EP 0 739 181 B1 (Plötzliche Herztod-Vorhersage), EP 0 906 058 B1 (Gerät zur Frequenzanalyse von Vorhofflimmern), DE 696 17 564 T2 (Taschenvorrichtung zur Detektion eines elektrischen, biologischen Signals, insbesondere eines elektrokardiographischen Signals).

Aus der EP-A-1 459 681 ist ein Verfahren bekannt, bei dem aus einem gemittelten EKG Signal u.a. die R-R-Abstände bestimmt werden und die erhaltenen Ergebnisse über ein Netzwerk an eine zentrale Workstation übertragen werden. Die DE-A-10 2006 002 045 beschreibt die Bestimmung von Frequenzbereichs-Analyseparametern für die Herzfrequenzvariabilität sowie eine Übertragung der EKG-Signale und der Parameter an eine externe Vorrichtung.

Bekanntlich stellt ein Elektrokardiogramm (EKG bzw. Herzstromkurve) in seiner allgemeinen Form den zeitlichen Verlauf der bioelektrischen Signale bzw. Potentialdifferenzen dar, die bei der Erregungsausbreitung und -rückbildung im Herzen entstehen. Daraus lassen sich u. a. direkte Hinweise auf Herzrhythmusstörungen bzw. indirekte Hinweise auf akute oder abgelaufene Herzinfarkte ableiten.

Neben der Abhängigkeit der (mittleren) Herzfrequenz vom Lebensalter ist aus der Physiologie bekannt, dass von der Aktivität des autonomen Nervensystems entscheidender Einfluss auf die Herzschlagfrequenz und auf den gesamten kardiovaskulären Zustand ausgeht. Während eine sympathische Stimulation bekanntlich über eine Freisetzung von Adrenalin und Noradrenalin die Schlagdauer reduziert, also die Frequenz erhöht, bewirkt der Parasympathikus überwiegend durch Freisetzung von Acetylcholin bekanntermaßen eine Herzfrequenzreduktion. Letzteres betrifft vor allem den Zustand der Körperruhe in liegender Position (vagale Stimulation).

Da die Herzstromkurve innerhalb einer Ableitung für jede Herzaktion ermittelt werden kann, andererseits unter einer Herzstromkurve die Registrierung der Erregungsabläufe der Vorhofe und Kammern verstanden wird und aus dieser Zeitfunktion zwecks Erfassung der Aktivität des autonomen Nervensystems untere Grenzfrequenzen von etwa 0,05 Hz erforderlich sind, wird die Aufgabe abgeleitet, die für das Herz-Kreislaufverhalten notwendigen kardiovaskulären Informationen aus der gesamten abgeleiteten EKG-Zeitfunktion einschließlich Überlagerungen zu bestimmen.

Zur Lösung der Aufgabe der Erfindung sei auf die Lehre der Ansprüche 1 bis 3 verwiesen.

Die Erfindung soll gemäß nachstehender Beschreibung sowie Ausführungsbeispielen und Figuren näher erläutert werden. Hierbei zeigen

Figur 1: Funktionsschema eines Tele-EKG.

Figur 2: Signalgemittelte EKG-Form aller während der EKG-Ableitezeit übereinander gelegten normalen ("periodischen") EKG-Intervalle am Beispiel eines 20jährigen herz-kreislauf-gesunden Probanden.

Figur 3: zeitlicher Ausschnitt eines 1kanaligen Tele-EKGs einschließlich markierte R-R-Abstände als zugehörige Herzperiodendauern.

Figur 4: Zeitlicher Ausschnitt von 1 Minute eines Tachogramms der Herzperiodendauern (R-R-Abstände) bzw. deren Reziprokwert Herzfrequenz:

a) 24jähriger Normalproband: deutlich bildet sich eine respiratorische Sinusarrhythmie mit den Atmungsperioden ab. Ersichtlich ist auch eine ausgeprägte Herzfrequenzvariabilität.

b) 72jährige Patientin mit koronarer 3-Gefäßerkrankung: neben der hohen mittleren Herzfrequenz von 94 min$^{-1}$ ist offensichtlich die Herzfrequenzvariabilität gering. Eine respiratorische Sinusarrhythmie bildet sich nicht aus. Am Ende des 60s-Tachogramms verändern sich die Herzperioden periodisch mit der anormalen Periode von 2 Herzschlägen, wobei die Änderungsamplituden gering sind.

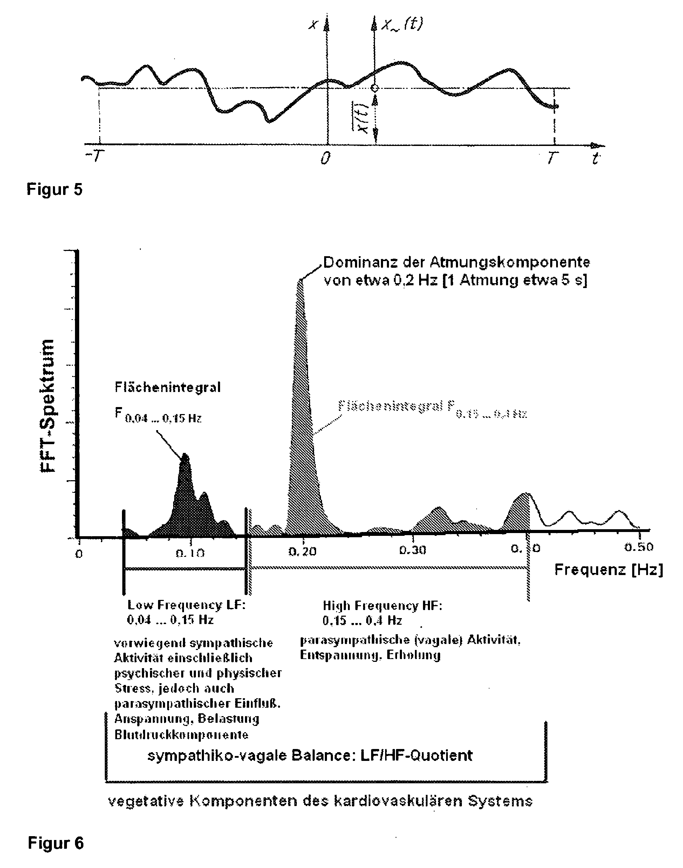

Figur 5: Signal x(t), zugehöriger linearer Mittelwert $\overline{x(t)}$ und Wechselkomponente x_(t).

Figur 6: Aus dem Tachogramm der Herzperiodendauern bei einem 20jährigen gesunden Probanden abgeleitetes FFT-Spektrum und einer 3minutigen Ableitungszeit [nach Baumert, J-H, A W Frey und M Adt: Analyse der Herzfrequenzvariabilität. Grundlagen, Methodik und mögliche Anwendung in der Anästhesie. Anaestesist 44 (1995), 677-686] einschließlich Frequenzbereichscharakteristika [nach: Heart rate variability. Standards of measurement, physiological interpretation and clinical use. European Heart Journal (1996) 17, 354-381].

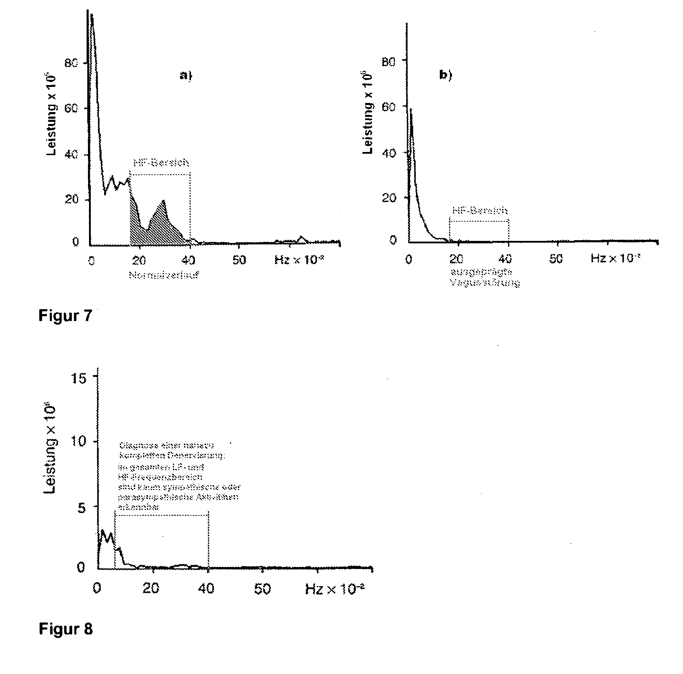

Figur 7: Spektrale Leistungsdichte der Herzfrequenzvariation in Ruhe bei liegenden Personen und einer EKG-Messzeit von 5 Minuten:

a) 35jähriger Normalproband: in allen 3 Bereichen liegen die Frequenzkomponenten in den (hier nicht angegebenen) Normgrenzen

b) 35jähriger TypI-Diabetiker mit somatoviszeraler Polyneuropathie mäßigen Grades: die Unterschiede im LF- und HF-Bereich sind beträchtlich, vor allem im HF-Bereich.

Figur 8: Spektrale Leistungsdichte der Herzfrequenzvariation in Ruhe bei einem liegenden 37jährigen Patienten mit Guillain-Barré-Strohl-Polyradikuloneuritis. Es liegt eine extreme Leistungsreduzierung in beiden Frequenzbereichen vor (beachte: empfindlicherer Maßstab als in Figur 7 verwendet).

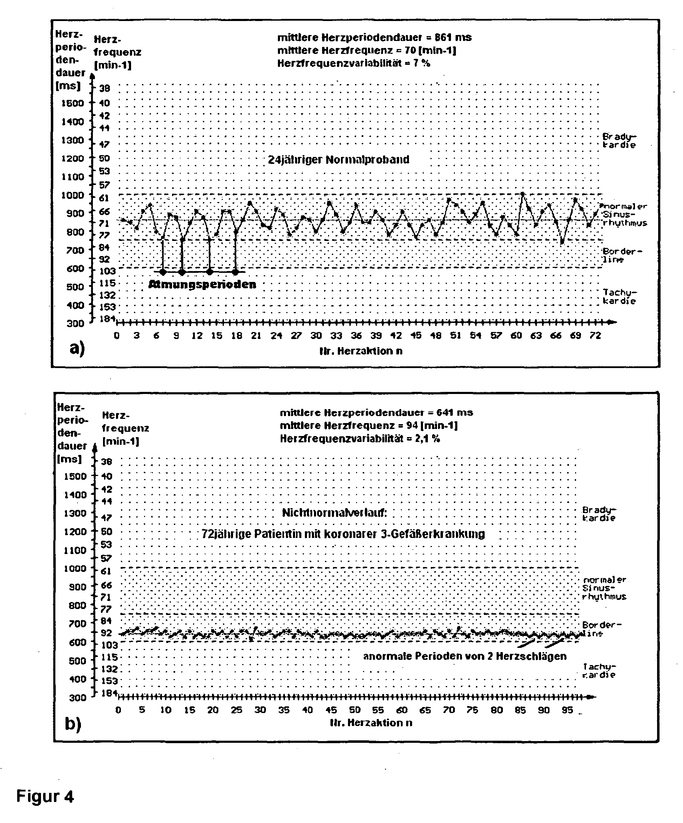

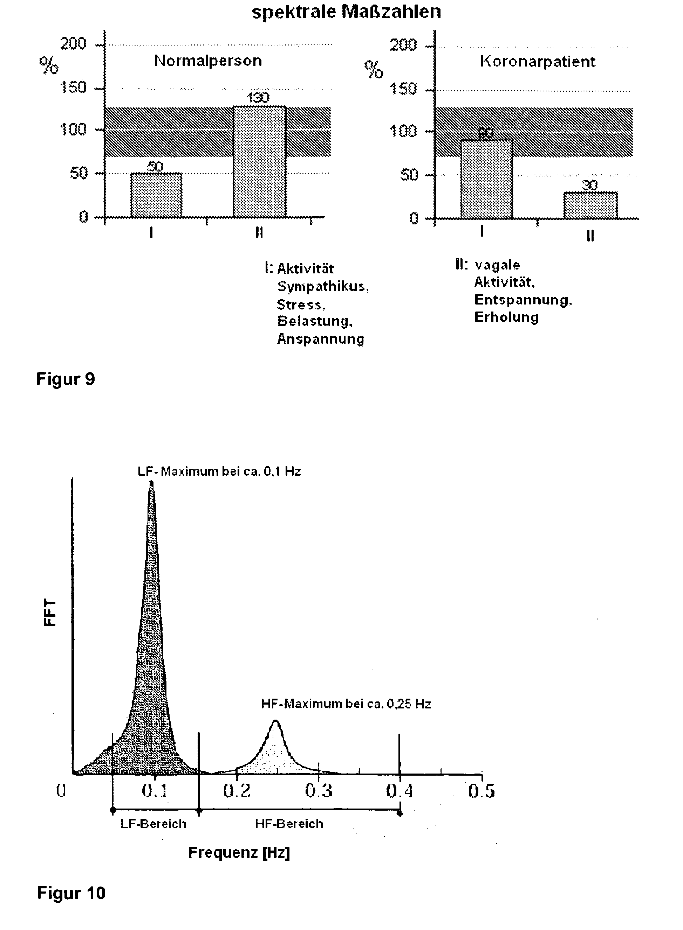

Figur 9: Charakteristische Beispiele für spektrale Maßzahlen: links: für einen Normalprobanden entsprechend Figur 4a), rechts: für einen Koronarpatienten nach Figur 4b).

Figur 10: Glättung eines aus dem Tachogramm der Herzperiodendauern abgeleiteten FFT-Spektrums bei einer 28jährigen liegenden Normalperson.

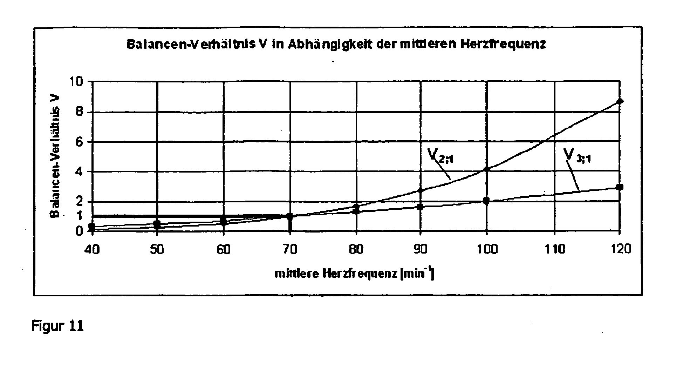

Figur 11: Abhängigkeit des definierten Balancen-Verhältnisses $V_{Balancen}$ von der mittleren Herzfrequenz $f_H$. Es ist ersichtlich, dass sich bei normaler Herzfrequenz von 70 $min^{-1}$ beide Wichtungen nicht unterscheiden, während niedrigere und höhere Herzfrequenzen zu deutlichen Unterschieden führen.

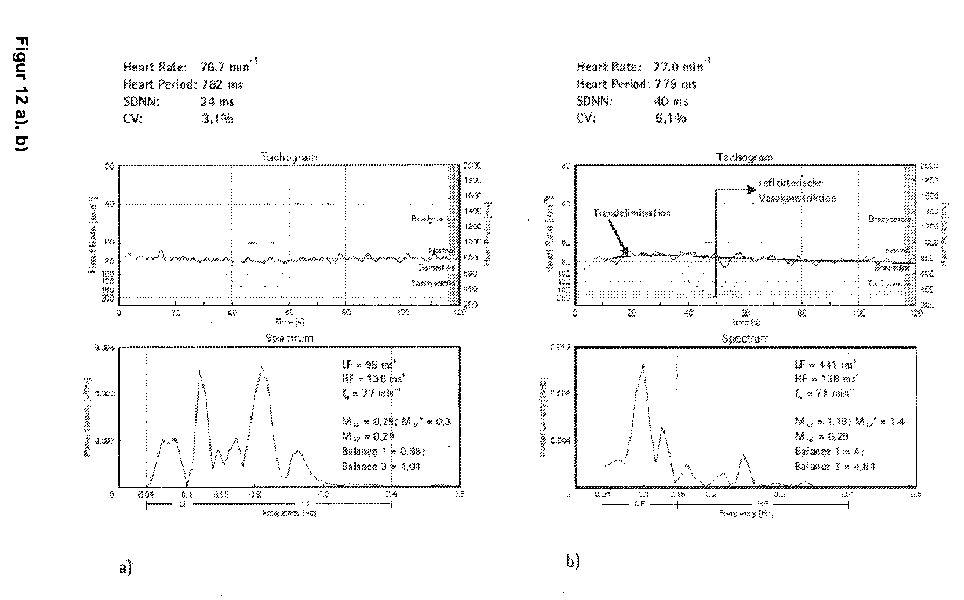

Figur 12a), b): Herzperiodendauer-Tachogramme und zugehörige FFT-Spektren sowie kardiovaskuläre Kennwerte, abgeleitet aus Tele-EKG bei einer 30jährigenden liegenden Versuchsperson (Raucher) unter Ruhe und reflektorischer Vasokonstriktion (passives plötzliches Eintauchen der linken Hand in Eiswasser). Es wird darauf verwiesen, dass die Power-Spektren unter a) und b) unterschiedliche Maßstäbe aufweisen [Figur a) mit 4mal höherer Empfindlichkeit].

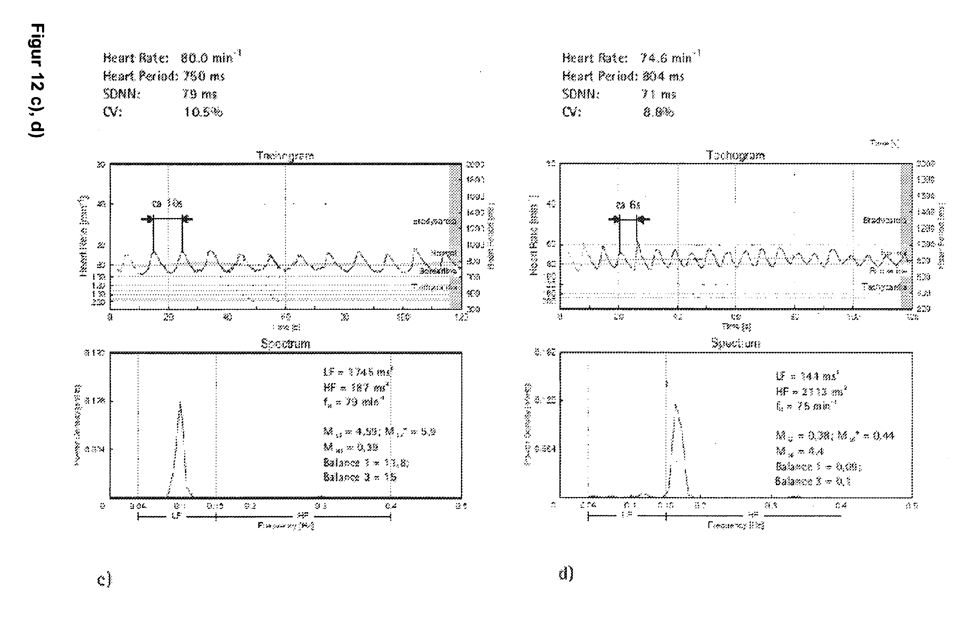

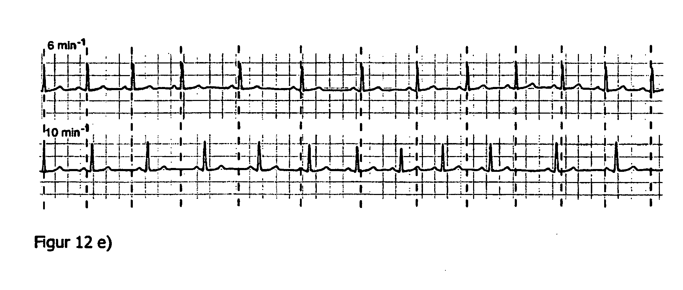

Figur 12 c), d): Herzperiodendauer-Tachogramme und zugehörige FFT-Spektren sowie kardiovaskuläre Kennwerte, abgeleitet aus Tele-EKG bei einer 30jährigenden liegenden Versuchsperson (Raucher) nach Figur 12a) unter vertiefter Brustatmung mit 6 und 10 min-1.

Figur 12 e): zeitlicher Ausschnitt aus den Tele-EKG bei der liegenden Versuchsperson nach den Figuren 12 c) und d) unter den vertieften Brustatmungen von 6 und 10 $min^{-1}$. Deutliche Unterschiede zeigen sich bei den R-R-Abständen sowohl beim Ein- als auch Ausatmen sowie bei den kardiovaskulären Kennwerten insgesamt [s. Figuren 12 c) und d)].

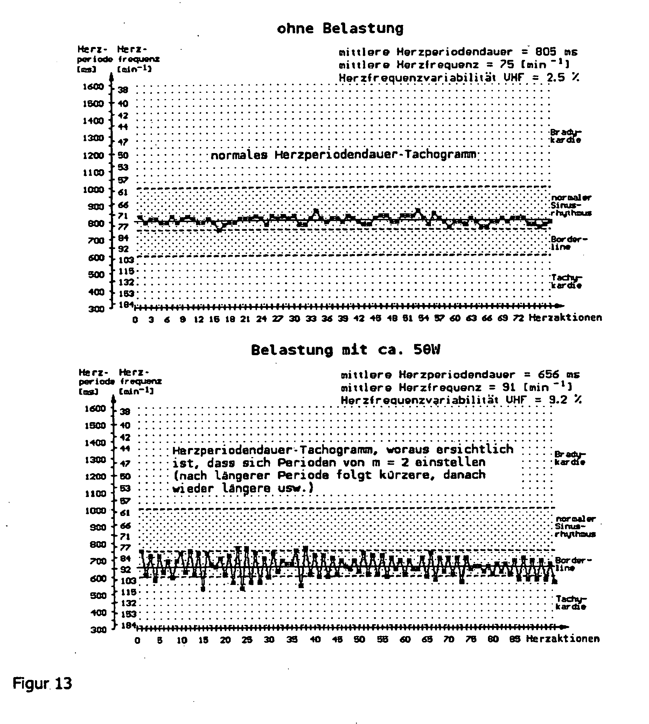

Figur 13: Verlauf der Herzperiodendauer-Tachogramme bei einem 68jährigen Herz-REHA-Patienten vor und unter Ergonometerbelastung. Bei einer Belastung von 50 W verändern sich die Herzperiodendauern z. T. wie ein elektronischer Multivibrator.

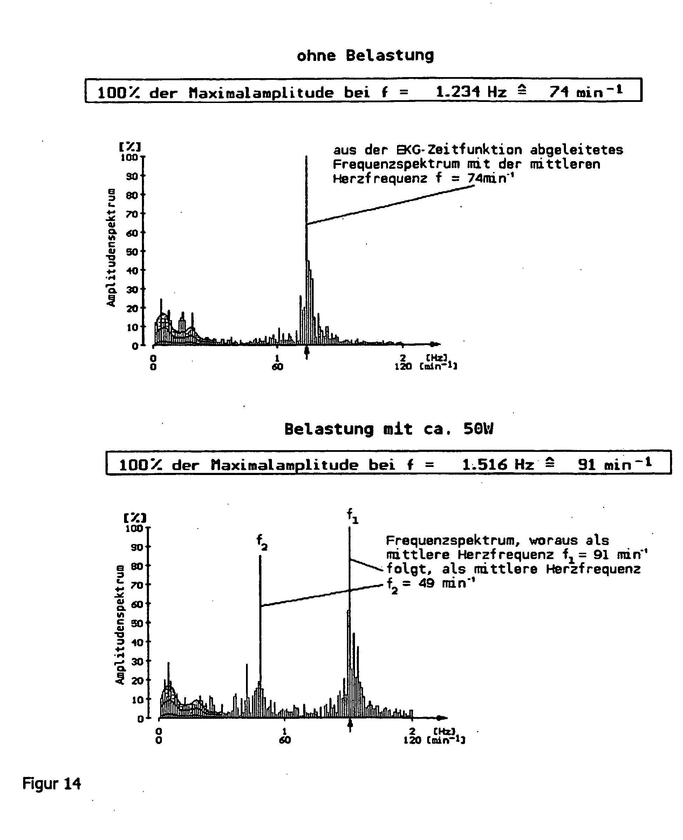

Figur 14: Amplitudenspektren, abgeleitet aus den zugehörigen EKG-Zeitfunktionen für die Versuchsperson und die Verhältnisse nach Figur 13.

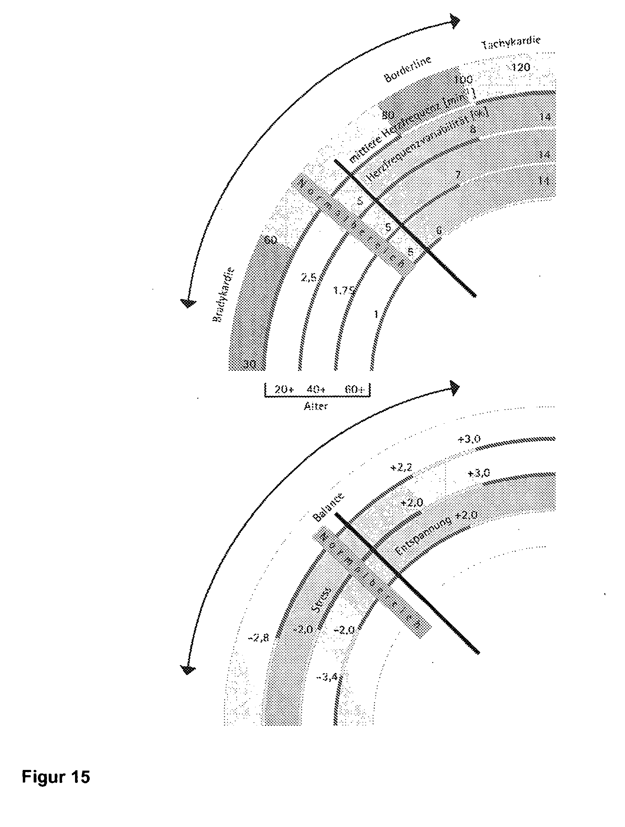

Figur 15: Tachometermäßige Oberflächengestaltung auf einem Display eines Mobilfunkgerätes bzw. Endgerät mittels telemedizinischen Datentransfers von kardiovaskulären Kennwerten. Als Variante ist dabei der Zeiger feststehend, während die Skalenteile sich entsprechend ihrer Werte veränderlich markieren.

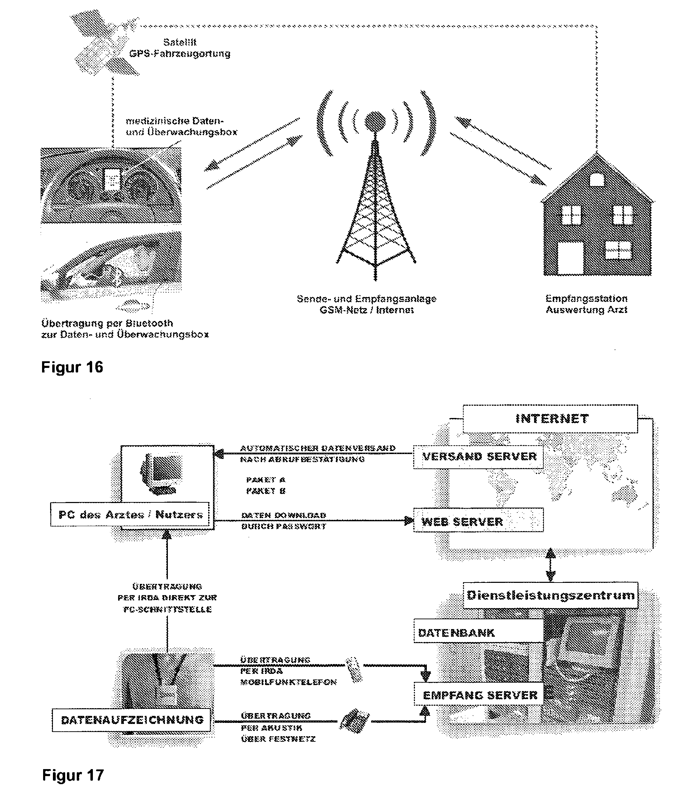

Figur 16: Ableitung und Erfassung von Vitaldaten im PKW oder LKW, insbesondere EKG, Blutdruck und Blutsauerstoffsättigung zu einer medizinischen Daten- und Überwachungsbox sowie nachfolgende telemedizinische Übertragung und Auswertung in einer Empfangsstation. Über Satellit ist eine ständige Fahrzeugortung und im Notfall auch Kommunikation möglich.

Figur 17: Kommunikationsprozeß von der EKG-Ableitung und Speicherung sowie des telemedizinischen Datentransfers unter Nutzung der verschiedensten analogen und digitalen Kommunikationswege.

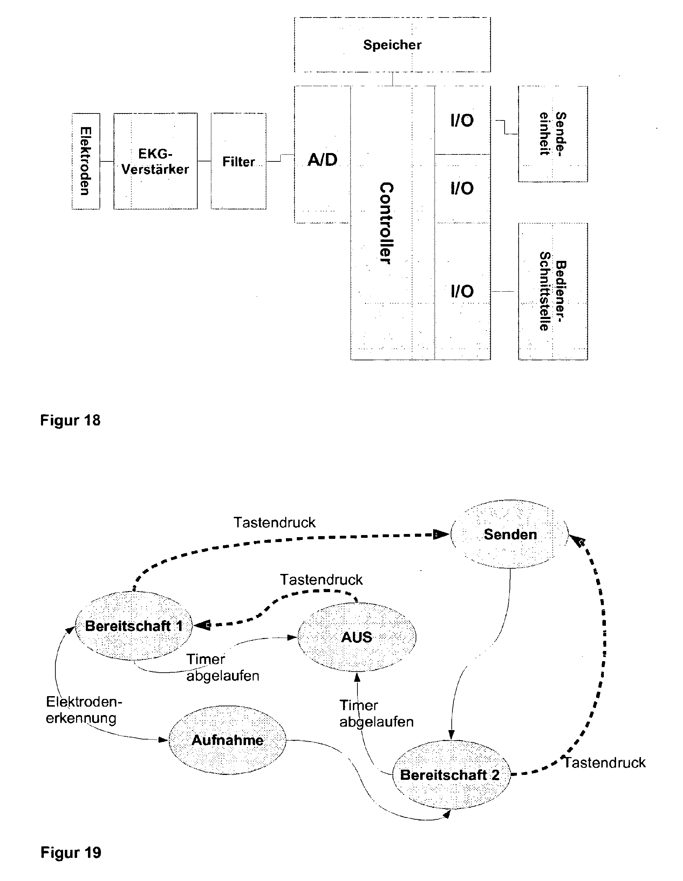

Figur 18: allgemeine Prinzipdarstellung eines telemedizinischen EKG-Sensors mit Signalverarbeitung und Speicherung sowie Bedienerschnittstelle und Sendeeinheit.

Figur 19: Automatengraph 1-Tasten-Bedienung.

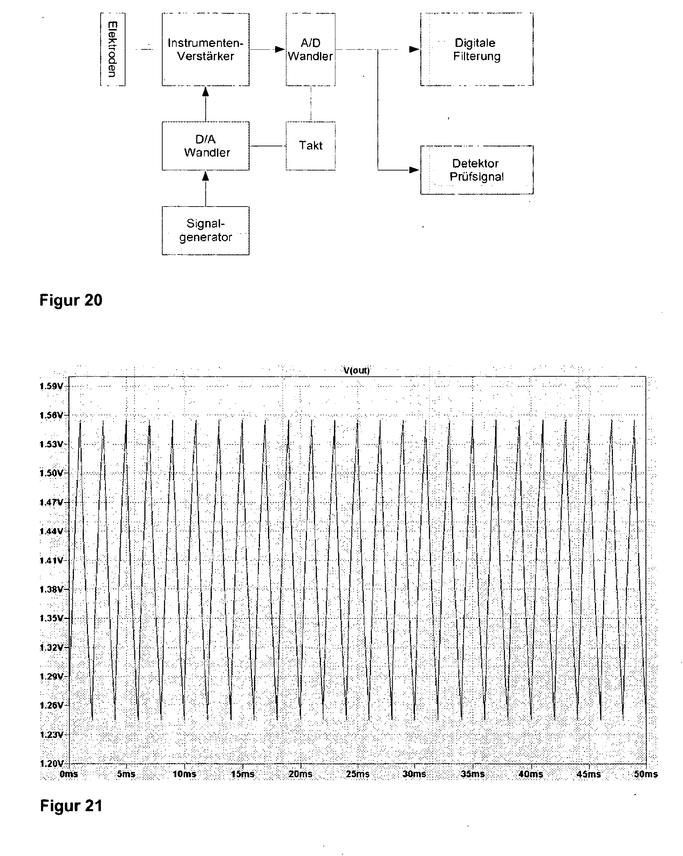

Figur 20: Blockschaltbild zur Amplitudenerkennung.

Figur 21: Prüfsignal (0,0013671875 mVss / 500 Hz) im Fall offene Elektroden.

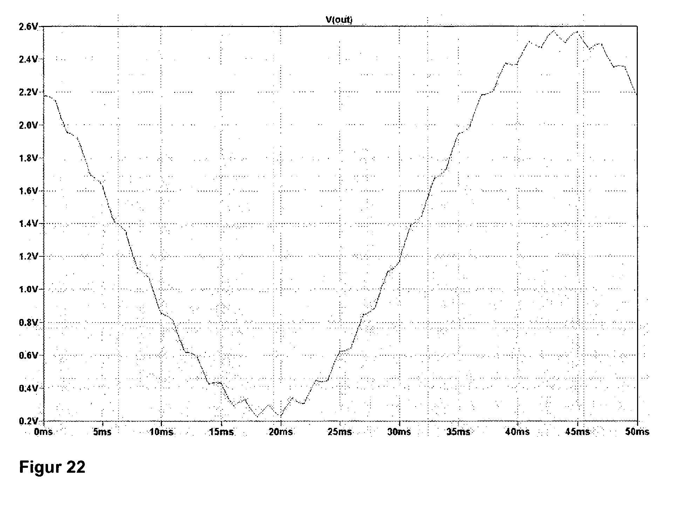

Figur 22: gedämpftes Prüfsignal mit überlagertem 20Hz-Nutzsignal 6 mVss im Fall angeschlossener Elektroden.

Wie aus der Signalanalyse bekannt ist, lassen sich aufgenommene Daten im

• Zeitbereich
• Frequenzbereich
• Amplitudenbereich

analysieren. Während im Zeitbereich das Verhalten der Messgröße in Abhängigkeit von der Zeit beschrieben wird, interessieren die im Signal enthaltenen Frequenzen mit der bekannten Diskreten Fourier-Transformation ebenso, wie die Datenanalyse im Amplitudenbereich. Typische Fragestellungen sind bei letzterem z. B. die Verteilung der Daten, Mittelwerte und Streuung.
In diesem komplexen Sinne werden erfindungsgemäß die abgeleiteten EKG-Signale analysiert, um die gestellte Aufgabenstellung nach Bestimmung von gezielten Herz-Kreislauf-Informationen aus der EKG-Zeitfunktion unter dem Aspekt einer telemetrischen Übertragung zu erfüllen. Dabei wird eine zeitdiskrete telemetrische EKG-Analyse vorgenommen.
Die Erfindung ist in den Ansprüchen definiert.
Im Mittelpunkt des Verfahrens zur Ableitung von Herz-Kreislauf-Informationen aus Herzstromkurven für telemedizinische Anwendungen stehen dynamische Prozesse, charakterisiert insbesondere durch die Erfassung und Bewertung der Herzperiodendauern (R-R-Abstände im EKG) innerhalb einer der telemetrischen Übertragung angepassten Mess- bzw. Ableitezeit.
So ist bekannt, dass sich bei Auswertung der Dauern der Herzaktionen, der sogenannten R-R-Abstände eines EKG, der Informationsgehalt gegenüber dem bei einer Formanalyse von EKG-Intervallen wesentlich erhöht. Das betrifft gleichfalls das telemedizinisch übertragene Elektrokardiogramm, das bisher lediglich meist qualitativ vor allem hinsichtlich auftretender Herz-Rhythmusstörungen bewertet wurde.
Aus einem abgeleiteten EKG wird als 1. Schritt das eigentliche EKG-Intervall, dessen Form, analysiert. Dazu werden alle während einer EKG-Ableitezeit von z. B. 2 min erkannten "Normal-EKG-Intervalle" (also ohne Extrasystolen usw.) übereinander gelegt und das mittlere Intervall, dessen Form, ermittelt und graphisch als "signalgemittelte EKG-Form" so dargestellt, dass ein Vergleich mit einem Normalverlauf möglich wird. Dabei werden aus einem abgeleiteten Tele-EKG nur Zeitwerte analysiert, d.h. keine Amplituden-Bewertungen vorgenommen. Damit kann sehr einfach und sofort Soll- mit Istverlauf verglichen werden, wie aus Figur 2 am Beispiel einer 20jährigen herz-kreislauf-gesunden Versuchsperson hervorgeht. Ebenso ist die die Abbildung der im zeitlichen Mittel auftretenden Spätpotentiale möglich, wobei Spätpotentiale bekanntlich hochfrequente, niederamplitudige Signale am Ende eines QRS-Komplexes sind, die ihren Ursprung in Regionen eines geschädigten Ventrikelmyokards mit verlangsamter Leitung haben. Da deren Amplitude im Oberflächen-EKG sehr gering ist, können Spätpotentiale nur bei hoher Verstärkung und mit speziellen Verfahren zur Rauschunterdrückung abgeleitet werden, was erfindungsgemäß möglich ist.
In einem 2. Schritt des Verfahrens werden aus dem abgeleiteten EKG die R-R-Abstände nach einem entsprechenden Erkennungsalgorithmus ermittelt (Figur 3) und sämtlich (also einschließlich Extraschläge) graphisch als Herzperiodendauer-Tachogramm dargestellt. Damit ist optisch sofort z. B. das Auftreten einer absoluten Arrhythmie und von allgemeinen Herz-Rhythmusstörungen ersichtlich.
Zur quantitativen Ermittlung der aus dem R-R-Tachogramm abzuleitenden Zeit- und Frequenzparameter wird zunächst ein Prüftest nach M auswertbaren ("normalen") R-R-Intervallen der vorliegenden Messzeit durchgeführt. Dabei wird von der üblichen Festlegung ausgegangen, dass vorzeitige Extraschläge (25 % Vorzeitigkeit bezogen auf das mittlere R-R-Intervall) und der postextrasystolische Schlag für die nachfolgenden Parameter-Bestimmungen im Zeit- und Frequenzbereich zu eliminieren und an den gleichen Zeitpunkten den aus allen Messungen zunächst bestimmten Herzperiodenmittelwert einzusetzen sind.
Nach einem solchen Vorgehen erfolgt eine Neuberechnung ("Präzisierung") der mittleren Herzperiodik aus den ermittelten M R-R-Abständen als bekannter arithmetischer Mittelwert der Herzperiodendauern $\overline{T_H}$ in [ms] wie folgt:

$$\overline{T_H} = \frac{1}{M} \sum_{\mu=1}^{M} T_\mu \quad [ms]$$

Daraus lässt sich die mittlere Herzfrequenz $\overline{f_H}$ ableiten, wobei als Umrechnung bekanntlich gilt
Herzfrequenz $f_H$ [Hz] = 1 / Herzperiodendauer $T_H$ [s] bzw.
Herzfrequenz $f_H$ [min$^{-1}$] = 60 / Herzperiodendauer $T_H$ [s].

Es soll im Tachogramm der Herzperiodendauern folgende bekannte kardiologische Einteilung der Herzfrequenz vorgenommen werden:

- Normaler Sinusrhythmus: 60 ... 80 min$^{-1}$ [Herzperiodendauer = 750 ... 1000 ms]
- Bradykardie: ≤ 60 min$^{-1}$ [Herzperiodendauer ≥ 1000 ms]
- Borderline: 80 ...100 min$^{-1}$ [Herzperiodendauer 600 ... 750 ms]
- Tachykardie: ≥ 100 min$^{-1}$ [Herzperiodendauer ≤ 600 ms].

Als absolute Abweichung der Herzperiodendauer $\Delta T_H$ zum Zeitpunkt der $\mu$-ten Herzaktion vom ermittelten arithmetischen Mittelwert $\overline{T_H}$ gilt entsprechend Figur 4

$$\Delta T_H(\mu) = T_H(\mu) - \overline{T_H}.$$

Daraus folgt für die "Standardabweichung" $s_{TH}$ bzw. die klinisch oft verwendete "absolute Herzfrequenzvariabilität":

$$s_{TH}\ [ms] = \sqrt{\frac{1}{M-1} \sum_{\mu=1}^{M} [T_H(\mu) - \overline{T_H}]^2}$$

Um z. B. den Einfluss der bekannten Tages-Nacht-Rhythmik zu minimieren, werden Relativwerte eingeführt, d.h. die absolute Variabilität der Herzperiodendauer $s_{TH}$ mit der Dimension "Sekunde", die Standardabweichung, wird auf die mittlere Herzperiodendauer der gleichen Dimension bezogen und zwecks Prozentangabe mit dem Faktor 100 multipliziert. Dieser kardiovaskuläre Kennwert stellt einen "Variationskoeffizienten" dar und wird abkürzend "(relative) Herzfrequenzvariabilität" VHF ($\Delta$HF) bzw. HRV genannt.

$$HRV\ [\%] = \frac{s_{TH}\ [ms]}{\overline{T_H}\ [ms]} 100\ [\%]$$

Dieser Variationskoeffizient, die (relative) Herzfrequenzvariabilität, ist also ein Mittelwert (im Sinne der Signaltheorie ein "Effektivwert") und - wie auch die vorn eingeführte mittlere Herzperiodik - abhängig vom Lebensalter: Mit steigendem Alter wird die Herzfrequenzvariabilität reduziert.

Im vorliegenden Fall der telemetrischen Anwendung wird auf die absoluten und relativen Standardabweichungen $s_{TH}$ bzw. HRV = VHF orientiert.

Die unter bestimmten physiologischen Bedingungen je Herzschlag sich einstellenden Änderungen der Herzperiodendauer sind bekanntlich Ausdruck der Aktivität des autonomen Nervensystems mit seinen Komponenten und zugleich sogenannten "Gegenspielern" Sympathikus und Parasympathikus. Es wird deshalb erfindungsgemäß nachfolgend die Aufgabe gestellt, aus einem Herzperiodendauer-Tachogramm die hierfür zugeordneten Informationen bzw. quantitative Maßzahlen einschließlich für "Stress" abzuleiten. Dabei ist physiologisch bekannt, daß zwischen körperlichen Stressreaktionen und dem autonomen Nervensystem ein unmittelbarer Zusammenhang besteht.

Stress ist ein medizinisches und psychologisches Phänomen, das in allen Altersgruppen und in allen sozialen Schichten vorkommt. Viele Menschen versuchen ihren Alltagsstress zu kompensieren. Ein sehr einfaches und wirkungsvolles Mittel zum Stressabbau ist bekanntlich die sportliche Betätigung. Im Körper angestauter Stress wird bei körperlicher Anstrengung abreagiert und verarbeitet, ohne dass der Gesundheit geschadet wird. Regelmäßiges, sinnvolles Sporttreiben hält den Körper fit, sorgt für neues Selbstbewusstsein und steigert die Abwehr gegen Stressreize. Das Herz-Kreislauf-System wird belastbarer.

Aus der Physiologie ist des weiteren bekannt, dass von der Aktivität des autonomen Nervensystems entscheidender Einfluss auf die Herzschlagfrequenz ausgeht. Während eine sympathische Stimulation bekanntlich über eine Freisetzung von Adrenalin und Noradrenalin die Schlagdauer reduziert, also die Frequenz erhöht, bewirkt der Parasympathikus überwiegend durch Freisetzung von Acetylcholin eine Herzfrequenzreduktion. Letzteres betrifft vor allem den Zustand der Körperruhe in liegender Position (vagale Stimulation).

Dieser Einfluss des autonomen Nervensystems durch die sich einstellende mittlere Herzfrequenz auf die Bewertung des kardiovaskulären Zustandes wird oft zu einseitig der vorn dargestellten "Herzfrequenzvariabilität" zugeordnet. Dies wird als entscheidender Mangel eingeschätzt, insbesondere auch bei einer Beurteilung von "Stress" bzw. "Belastung" oder des "Gegenspielers" Erholung. Mittels der allgemeinen Signaltheorie soll erfindungsgemäß gezeigt werden, dass ein erweitertes integrales quantitatives Maß hierfür ableitbar ist.

Aus der Signaltheorie ist bekannt, dass eine beliebige Zeitfunktion x(t) allgemein in den arithmetischen Mittelwert $\overline{x(t)}$ sowie in eine Wechselkomponente $x_\sim(t)$ zerlegbar ist (Figur 5):

$$x(t) = \overline{x(t)} + x_\sim(t)$$

Für den linearen (arithmetischen) Mittelwert von x(t) erhält man

$$\overline{x(t)} = \frac{1}{2T} \int_{-T}^{+T} x(t)\, dt.$$

$\overline{x(t)}$ entspricht bekanntlich direkt dem halben Wert des Fourierkoeffizienten $A_o$. Für periodische Signale ist zur Ermittlung von $\overline{x(t)}$ die Periodendauer T einzusetzen, für nichtperiodische Signale gilt

$$\overline{x(t)} = \lim_{T \to \infty} \frac{1}{2T} \int_{-T}^{T} x(t)\, dt.$$

Für den quadratischen Mittelwert folgt

$$\overline{x^2(t)} = \frac{1}{2T} \int_{-T}^{T} x^2(t)\, dt,$$

analog für nichtperiodische Signale

$$\overline{x^2(t)} = \lim_{T \to \infty} \frac{1}{2T} \int_{-T}^{T} x^2(t)\, dt.$$

Zwischen den Signalleistungen der Komponenten x(t), $\overline{x(t)}$ und $x_\sim(t)$ gilt folgender grundsätzliche Zusammenhang, der in der Mechanik als Steinerscher Satz bekannt ist:

$$\overline{x^2(t)} = [\overline{x(t)}]^2 + \overline{x_\sim^2(t)}$$

Die Gesamtleistung eines Signals setzt sich demnach zusammen aus dem Quadrat des linearen (arithmetischen) Mittelwertes sowie der mittleren Leistung der Wechselkomponente, die dem Quadrat des Effektivwertes $x_{eff}$ entspricht:

$$x_{eff} = \sqrt{\overline{x^2(t)}}$$

Dieser Satz von Steiner lässt sich erfindungsgemäß auf den "Herzperiodendauer-Tachogrammverlauf R-R-Abstand" anwenden. Da jedoch bezüglich der Zeit t nur nach jeder Herzaktion $\mu$ ein Messwert auftritt, wird anstelle x (t) der vorn eingeführte Terminus $T_H(\mu)$ den nachfolgenden Betrachtungen zugrunde gelegt. Dieses Gesamtsignal $T_H$ (p) setzt sich, wie bereits begründet wurde, aus der Addition des arithmetischen Mittelwertes $\overline{T_H}$ und der absoluten Abweichung der Herzperiodendauer $\Delta T_H$ zum Zeitpunkt der $\mu$-ten Herzaktion vom Mittelwert zusammen:

$$T_H(\mu) = \overline{T_H} + \Delta T_H(\mu)$$

Analog zum vorn dargelegten Steinerschen Satz der Signaltheorie gilt erfindungsgemäß folglich gleichwertig für die Herzperiodendauer-Analyse

$$\overline{T^2_H(\mu)} = [\overline{T_H}]^2 + [\overline{\Delta T_H(\mu)}]^2 = [\overline{T_H}]^2 + [s_{TH}]^2.$$

Anders ausgedrückt:

Die Gesamtsignalleistung, die aus dem R-R-Signal ermittelt werden kann, entspricht der Summe aus Signalleistung aus dem arithmetischen Signal (der mittleren Herzfrequenz zum Quadrat) sowie dem Quadrat $s_{TH}^2$ der Standardabweichung. Das gilt analog auch für die im R-R-Signal enthaltene Information unter Anwendung der Informationstheorie.

Letztere Gleichung für die Gesamtsignalleistung lässt sich normieren: Division durch $[\overline{T_H}]^2$ bzw. gleichwertig Multiplikation mit $[\overline{f_H}]^2$, so dass sich erfindungsgemäß ergibt:

$$\frac{\overline{T^2_H(\mu)}}{[\overline{T_H}]^2} = 1 + \frac{[s_{TH}]^2}{[\overline{T_H}]^2} \qquad bzw.$$

$$\overline{T^2_H(\mu)} \cdot [\overline{f_H}]^2 = 1 + [s_{TH}]^2 \cdot [\overline{f_H}]^2$$

Aus dieser Darstellung geht hervor, dass die normierte R-R-Gesamtsignalleistung nur variierbar ist durch die gewichtete Komponente

$$[s_{TH}]^2 \cdot [\overline{f_H}]^2.$$

also durch Wichtung des Quadrates $s_{TH}^2$ der Standardabweichung, der Herzfrequenzvariabilität, mit dem Quadrat der mittleren Herzfrequenz $\overline{f_H}$. Dies entspricht dem Quadrat der relativen Herzfrequenzvariabilität (Variationskoeffizient) VHF ($\Delta$HF) bzw. HRV.

Eine Beschreibung des kardiovaskulären Zustandes allein durch die Standardabweichung $s_{TH}$ bzw. die absolute Herzfrequenzvariabilität ist nicht ausreichend. Mit dem Wichtungsfaktor (mittlere) Herzfrequenz zum Quadrat - letztere wird durch das autonome Nervensystem mit seinen Komponenten Sympathikus und Parasympathikus entscheidend geprägt - wird der kardiovaskuläre Zustand erfindungsgemäß differenziert bewertbar. Dies wird auch aus den nachfolgenden Darstellungen der FFT-Analyse der Signal-Wechselkomponente, der absoluten Abweichung der Herzperiodendauer zum Zeitpunkt der $\mu$-ten Herzaktion vom arithmetischen Mittelwert, deutlich.

Wird die eingeführte absolute Abweichung der Herzperiodendauer zum Zeitpunkt der $\mu$-ten Herzaktion vom ermittelten arithmetischen Mittelwert $\overline{T_H}$

$$\Delta T_H(\mu) = T_H(\mu) - \overline{T_H}$$

nicht einer zeitlichen Mittelung, sondern einer schnellen FourierTransformation (FFT) unterzogen, so erhält man das bekannte "(Leistungs-)Spektrum der Herzfrequenzvariabilität", präziser formuliert "(Leistungs-)Spektrum der absoluten Abweichungen der Herzperiodendauer vom Mittelwert $\overline{T_H}$".

Figur 6 zeigt ein aus einem Herzperiodendauer-Tachogramm einer 20jährigen herzkreislauf-gesunden Person abgeleitetes FFT-Spektrum, wobei die Ableitungszeit 3 Minuten betrug. Aus physikalischmathematischen Gründen ist hierfür der Bereich bis 0,5 Hz zugrunde zu legen. Da eine solche Frequenzanalyse lediglich für die Wechselkomponente $\Delta T_H(\mu)$ [der absoluten Abweichung der Herzperiodendauer vom Mittelwert $\overline{T_H}$] durchgeführt wird und diese keinen Gleichanteil besitzt, beträgt hierfür der Wert bei f = 0 Hz stets Null. Das Gesamtsignal $T_H(\mu)$ besitzt jedoch den bereits diskutierten Gleichanteil, den arithmetischen Mittelwert $\overline{T_H}$.

Bisher liegt kein einheitlicher Standard zur "Herzfrequenzvariabilitätsanalyse FFT-Spektrum" vor, jedoch eine große Vielfalt von Ergebnissen und Arbeiten. Immer öfter wird bezüglich der Spektren-Analysen auf die Richtlinien der Task Force of European Society of Cardiology vom Jahr 1996 sowie der "North American Society of Pacing and Electrophysiology" verwiesen [Heart rate variability. Standards of measurement, physiological interpretation and clinical use. European Heart Journal (1996) 17, 354-381]. Darauf beruht auch die grundlegende Arbeit von Bürklein, M, Vogt L und W Banzer: Messverfahren zur Erfassung der Herzfrequenzvariabilität - Eine vergleichende Studie. Cross validation of heart rate variability measurements before and after exercise. Deutsche Zeitschrift für Sportmedizin. Jahrgang 56, Nr. 12 (2005).

Die Task-Richtlinie sowie die Arbeit von Bürklein sind die Grundlage für eine FFT-Analyse, wie auch aus Figur 6 hervorgeht. Hier sind die typischen Frequenzbereiche für den Fall einer sogenannten "Kurzzeitanalyse" von etwa 2 bis 5 min EKG-Ableitungszeit entsprechend Task-Richtlinie gekennzeichnet und charakterisiert. Daraus geht der jeweilige Einfluß der vegetativen Komponenten des kardiovaskulären Systems hervor:

- Frequenzbereich "Low Frequency" 0,04 bis 0,15 Hz: Diesem Bereich ist eine vorwiegend sympathische kardiovaskuläre Aktivität zuordenbar, damit gleichfalls ein auftretender psychischer und physischer Stress. In der Literatur wird dargestellt, dass sich jedoch ebenso eine parasympathische Komponente hier abbilden kann. Wie die Physiologie lehrt, sind diesem Bereich auch die Traube-Hering-Schwingungen als Ausdruck für die auftretenden Blutdruckperiodika enthalten. In der Literatur wird eine allgemeine "Anspannung" bzw. "Belastung" diesem Low-Frequency-Frequenzbereich zugeordnet.
- Frequenzbereich "High Frequency" 0,15 bis 0,4 Hz: typisch hierfür ist die auftretende parasympathische (vagale) Aktivität, damit atemsynchrone Herzfrequenzfluktuationen der respiratorischen Sinusarrhythmie [Horn, A: Diagnostik der Herzfrequenzvariabilität in der Sportmedizin - Rahmenbedingungen und methodische Grundlagen. Diss, Fakultät für Sportwissenschaft, Ruhr-Universität Bochum 2003].
- Das spektrale Flächenverhältnis zwischen der LF- und HF-Komponente wird als sympathovagale Balance bezeichnet. Dabei ist jedoch zu beachten, dass eine Veränderung in der LF-Komponente - wie oben erwähnt - sowohl sympathisch als auch parasympathisch vermittelt werden kann. Diese Erfassung und Quantifizierung stellt ein bisher nicht gelöstes Problem auch bei der sympathovagalen Balance dar.

Die in Figur 6 nicht markierte niederfrequente Frequenz-Komponente von etwa 0,01 - 0,05 Hz beschreibt u.a. den thermoregulatorischen Einfluss. In der Literatur gibt es hierzu noch sehr unscharfe Darstellungen. Da eine solche Frequenz-Analyse und -bewertung im Sinne des bekannten Abtasttheorems der Signaltheorie wesentlich längere EKG-Ableitungszeiten als im vorliegenden Fall der telemedizinischen Anwendung erfordern würde, muss dieser Bereich hierfür ausgeschlossen werden. Es erfolgt deshalb eine FFT-Spektrums-Analyse nur für den charakterisierten Frequenzbereich von 0,04 bis 0,4 Hz, um vor allem auch den für das kardiovaskuläre System so wesentlichen vegetativen Einfluss zu quantifizieren. Im Sinne des erweiterten Abtasttheorems sollte hierfür als Mindest-Analysedauer bei einer Frequenz von 0,04 Hz das ca. 5 bis 10-fache der aus dem Theorem folgenden Zeit zugrunde gelegt werden, also ca. 120 Sekunden.

Methodische Voraussetzungen für die Durchführung einer FFT-Analyse sind u.a. Stationarität und Störungsfreiheit der R-R-Aufzeichnung, die in einer vorbereiteten Aufarbeitung der R-R-Reihe (Trendelimination, Filterung von Artefakten, Resampling u. a.) sicherzustellen sind. Auf Grund der Stationaritätsforderung ist hierbei eine solche Frequenzanalyse nur für kurze Aufzeichnungsdauern von 2 bis 5 min - "Kurzzeitanalyse" - sinnvoll. Dies entspricht dem Wert, der oben aus dem erweiterten Abtasttheorem abgeleitet wurde und für telemetrische Anwendungen sich als zweckmäßig erweist. Voraussetzung für die Ableitung von Normalwerten ist, dass das EKG im liegenden Zustand abgeleitet wird und mit der Messung erst dann begonnen wird, wenn sich das Kreislauf-System im stationären Zustand befindet. Dies ist nach ca. 2 Minuten mit ausreichender Näherung der Fall.

In den Figuren 7 und 8 sind Ausführungsbeispiele typischer klinischer Verläufe von FFT-Leistungsdichtespektren der absoluten Abweichungen der Herzperiodendauer vom Mittelwert $\overline{T_H}$ dargestellt, wobei ein Leistungsspektrum bekanntlich dem quadrierten FFT-Amplitudenspektrum entspricht.

So zeigt Figur 7 den gegenüberstellenden Verlauf der spektralen Leistungsdichten bei einem 35jährigen liegenden Gesunden sowie bei einem gleichaltrigen TypI-Diabetiker mit somatoviszeraler Polyneuropathie mäßigen Grades, dabei bei einer EKG-Messzeit von jeweils 5 Minuten. Auf Grund der längeren Ableitungszeit ist es zusätzlich näherungsweise möglich, den niederfrequenten Bereich 0,01 ... 0,04 Hz auszuwerten. Während sich die Komponenten in diesem Bereich nur wenig unterscheiden, sind die Unterschiede in den beiden anderen Bereichen sehr deutlich. Vor allem im rein vagal bedingten HF-Band ist die spektrale Leistung extrem erniedrigt, so dass die Diagnose einer ausgeprägten Vagusstörung bei erhaltener sympathischer kardialer Innervation gestellt werden musste.

Figur 8 zeigt als weiteres Ausführungsbeispiel das Spektrum bei einem 37jährigen Patienten mit Guillain-Barre-Strohl-Polyradikuloneuritis auf dem Höhepunkt der Erkrankung. Die Anteile in allen Frequenzbereichen sind extrem erniedrigt, wobei der Ordinaten-Maßstab im Vergleich zu Figur 7 wesentlich empfindlicher eingestellt wurde, um die Komponenten noch darstellen zu können. Es wurde die Diagnose einer nahezu kompletten kardialen Denervierung gestellt.

Wie aus den Verläufen der spektralen Leistungsdichten in den Figuren 7 und 8 hervorgeht, lassen sich qualitativ die Amplitudenanteile in den festgelegten Frequenzbereichen vergleichen. Neben Normalverläufen zeigen sich typische Nichtnormalverläufe. Erfindungsgemäß wird daher eine Quantifizierung vorgenommen.

Ausgehend von abzuleitenden Normalwerten für die bei den diskreten Frequenzen $nf_A$ [$f_A$ = Abtastfrequenz] auftretenden spektralen Leistungs-Normal-Amplituden $A(nf_A)$ in den Frequenzbereichen 0,04...0,4 Hz werden zugehörige Flächenintegrale (Summen) F bei einer EKG-Ableitezeit für eine Messung von 120 Sekunden bei Normalprobanden wie folgt bestimmt (s. Figur 6):

- LF-Bereich 0,04...0,15 Hz:

$$F_{normal\ 0,04...0,15\ Hz} = \Sigma\, A(nf_A)$$

- HF-Bereich 0,15...0,4 Hz:

$$F_{normal\ 0,15...0,4\ Hz} = \Sigma\, A(nf_A)$$

Von diesen "Normal-Flächensummen" wird erfindungsgemäß jeweils der mittlere Normalbereich für diese Frequenzbereiche bestimmt. Falls Normalverteilung vorliegt gilt bekanntlich: Mittelwert $\pm$ Standardabweichung.

Wird nun ein beliebiges Spektrum abgeleitet, so wird erfindungsgemäß jeweils die Flächensumme $F_\lambda$ für die angegebenen Frequenzbereiche gebildet und auf den zugehörigen Flächennormalwert bezogen. Diese relativen Werte werden "spektrale Maßzahlen M" genannt:

$$M_{LF} = \frac{F_{0,04\ ...\ 0,15\ Hz}}{mittlerer\ Flächen - Normalwert}\,100\,[\%]$$

$$M_{HF} = \frac{F_{0,15\ ...\ 0,4\ Hz}}{mittlerer\ Flächen - Normalwert}\,100\,[\%]$$

Da diese spektrale Maßzahlen stets auf die Normalwerte des entsprechenden Frequenzbereiches bezogen sind, ist sofort ersichtlich, ob autonome Dysbalancen auftreten.

In Figur 9 sind als Ausführungsbeispiele 2 typische Fälle für spektrale Maßzahlen dargestellt, die sich aus ermittelten Herzperiodendauer-Tachogrammen (R-R-Abstände) eines Tele-EKG ableiten lassen:

- Normalproband: kaum Stresszustand, guter Erholungszustand mit ausgeprägter respiratorischer Sinusarrhythmie (analog nach Figur 4a)),
- Koronarpatient mit geringer Erholungskomponente (entsprechend Figur 4b).

Aus den FFT-Spektren nach den Figuren 6 bis 8 folgt, dass der nach den Richtlinien der Task Force of European Society of Cardiology sowie der North American Society of Pacing and Electrophysiology vom Jahr 1996 definierte Auswerte-Frequenzbereich von 0,04 bis 0,4 Hz die Aktivität der vegetativen Komponenten des kardiovaskulären Systems dominant bestimmt und im folgenden erfindungsgemäß im Sinne der Signaltheorie tiefer zu analysieren ist, während der den thermoregulatorischen Einfluss charakterisierende Frequenzbereich von etwa 0,01 bis 0,04 Hz wegen der erforderlichen längeren EKG-Ableitungszeiten für die vorliegende telemedizinische Anwendung nicht betrachtet werden soll. Des weiteren wird den folgenden Betrachtungen eine entsprechende Trendkorrektur zugrunde gelegt.

Folglich kann mit ausreichender Näherung gesetzt werden, dass die spektralen FFT-Leistungsdichtespektrum-Flächen des definierten Frequenzbereiches von 0,04 bis 0,4 Hz dem Quadrat der Standardbweichung $s_{TH}{}^*$ entsprechen, wobei $s_{TH}{}^*$ im Gegensatz zu $s_{TH}$ nur den Teil der Standardabweichung darstellt, der durch die beiden vegetativen Komponenten des autonomen Nervensystems bestimmt wird.

Es lässt sich erfindungsgemäß setzen

$$\int_{0,04}^{0,4\,Hz} Leistungsdichtespektrum \approx \left[s_{TH}{}^*\right]^2 = \left[s_{TH\,vegetativ}\right]^2$$

bzw.

$$\int_{0,04}^{0,15\,Hz} Leistungsdichtespektrum \; + \; \int_{0,15}^{0,4\,Hz} Leistungsdichtespektrum \approx \left[s_{TH\,vegetativ}\right]^2$$

Demnach kann die Herzfrequenzvariabilität $s_{TH}{}^*$, die den beiden Komponenten des autonomen Nervensystems, dem Sympathikus und Parasympathikus, zuzuordnen ist, aus den beiden Flächen-Komponenten $F_{0,04...0,15\,Hz}$ sowie $F_{0,15...0,4\,Hz}$ bestimmt werden.

Es wurde bereits gezeigt, dass eine quantitative Beschreibung des kardiovaskulären Zustandes im Sinne des Steinerschen Satzes der Signaltheorie allein durch $s_{TH}{}^2$ und somit auch durch das Leistungsdichtespektrum nicht ausreichend ist, jedoch durch Einführung des Wichtungsfaktors "(mittlere) Herzfrequenz zum Quadrat" der kardiovaskuläre Zustand bezüglich des Einflusses des autonomen Nervensystems beschreib- und steuerbarer wird. Somit folgt:

$$\left[\overline{f_H}\right]^2 \int_{0,04}^{0,4\,Hz} Leistungsdichtespektrum \approx \left[\overline{f_H}\right]^2 \left[s_{TH\,vegetativ}\right]^2$$

bzw.

$$\left[\overline{f_H}\right]^2 \int_{0,04}^{0,15\,Hz} Leistungsdichtespektrum + \left[\overline{f_H}\right]^2 \int_{0,15}^{0,4\,Hz} Leistungsdichtespektrum \approx \left[\overline{f_H}\right]^2 \left[s_{TH\,vegetativ}\right]^2 .$$

Setzt man abkürzend

$$\int_{0.04}^{0.15\,Hz} Leistungsdichtespektrum = F_{0,04\ldots0,15\,Hz} = A$$

$$\int_{0.15}^{0.4\,Hz} Leistungsdichtespektrum = F_{0,15\ldots0,4\,Hz} = B,$$

so erhält man erfindungsgemäß für die gewichteten Summanden

$$[\overline{f_H}]^2\,A + [\overline{f_H}]^2\,B \approx [\overline{f_H}]^2\,[s_{TH\,vegetativ}]^2.$$

Also lassen sich als Wichtungen definieren:

$$[\overline{f_H}]^2\,A \quad sowie \quad [\overline{f_H}]^2\,B$$

Ist z. B. der Summand A bereits größer als normal, so wird er durch die Wichtung mit dem Quadrat von $\overline{f_H}$ im Falle einer tachykarden Herzfrequenz beträchtlich vergrößert. Dies gilt zwar ebenso für den Summanden B, jedoch wird dieser möglicherweise wesentlich kleiner als A sein, weil dann eine respiratorische Sinusarrhythmie kaum vorhanden ist.
Folglich lassen sich mit dieser Wichtung folgende Aktivitätsmaße des autonomen Nervensystems bezüglich des kardiovaskulären Systems definieren:

Maß für sympathische Aktivität, Stress, Belastung $\sim [\overline{f_H}]^2 A$

Maß für parasympathische Aktivität, Entspannung und Erholung $\sim [\overline{f_H}]^2 B$

Mit einem solchen Vorgehen wird stets das gesamte R-R-Signal in der Wirkung auf das kardiovaskuläre System analysiert und nicht nur die Wechselkomponente $\Delta T_H\,(\mu)$. Das gilt auch für die nachfolgenden Betrachtungen zu weiteren Wichtungsvarianten.
Während eine sympathische Aktivität die Herzfrequenz erhöht, wird sie durch eine parasympathische erniedrigt. Dies kann erfindungsgemäß quantitativ durch folgende Wichtung 2 realisiert werden:

$$[\overline{f_H}]^2\,A \quad sowie \quad \frac{B}{[\overline{f_H}]^2}.$$

Damit kann der sympathische im Vergleich zum parasympathischen Aktivitätsanteil der LF-Komponente rechnerisch noch stärker als bei der 1. Wichtung angehoben werden. Für die Aktivitätsmaße des autonomen Nervensystems erhält man analog zur Wichtung 1:

Maß für sympathische Aktivität, Stress, Belastung $\sim [\overline{f_H}]^2 A$

Maß für parasympathische Aktivität, Entspannung und Erholung $\sim \dfrac{B}{[\overline{f_H}]^2}$

Da der HF-Bereich der FFT bereits ausschließlich die parasympathische (vagale) Aktivität des autonomen Nervensystems charakterisiert, ist eine Wichtung dieses Bereiches im Sinne der Physiologie nicht erforderlich, so dass als 3. Wichtungsvariante definiert werden kann:

$$[\overline{f_H}]^2 \, A \quad sowie \ B \, .$$

Folglich gilt hierfür

Maß für sympathische Aktivität, Stress, Belastung $\sim [\overline{f_H}]^2 A$
Maß für parasympathische Aktivität, Entspannung und Erholung - B.

Es wurde dargestellt, dass das spektrale Flächenverhältnis zwischen der LF- und HF-Komponente in der Literatur als sympatho-vagale Balance bezeichnet wird. Für die eingeführten Wichtungen erhält man:

• Wichtung 1:

Mit

$$[\overline{f_H}]^2 \, A + [\overline{f_H}]^2 \, B \approx [\overline{f_H}]^2 \, [s_{TH\,vegetativ}]^2$$

stellt sich erfindungsgemäß die Balance als Verhältnis A / B dar, so dass eine solche Wichtung die Balance nicht verändert. Dies stellt dann ein Problem dar, wenn die spektralen Frequenzkomponenten im LF-Bereich hauptsächlich vagal bedingt sind. Das hätte zur Folge, dass sich dies fälschlicherweise als gestörte Balance darstellen würde.

• Wichtung 2:

$$sympathiko - vagale\ Balance = \frac{A}{B}\,[\overline{f_H}]^4 \, .$$

Daraus folgt, dass eine erhöhte Herzfrequenz zu einer beträchtlichen Störung der sympatho-vagalen Balance führt (Anstieg mit der 4. Potenz von $\overline{f_H}$), was sich auch physiologisch als richtige Zielstellung erweist. Ist folglich die LF-Komponente erhöht und die durch getrennte Summandenichtung erfindungsgemäß eingeführte sympatho-vagale Balance im gleichen Sinne, so kann nur eine erhöhte Sympathikusaktivität die Ursache sein und nicht der Parasympathikus.

• Wichtung 3:

Analog gilt:

$$sympathiko - vagale\ Balance = \frac{A}{B}\,[\ \overline{f_H}]^2 \, .$$

Eine erhöhte Herzfrequenz führt auch bei dieser Wichtung zu einem Anstieg der sympatho-vagalen Balance, dabei mit der 2. Potenz von $\overline{f_H}$ entsprechend der allgemeinen Definition der Signalleistung. Deshalb wird diese Wichtung als Vorzugsvariante zugrunde gelegt.

Figur 10 zeigt ein aus dem Tachogramm der Herzperiodendauern abgeleitetes und geglättetes FFT-Spektrum bei einer 28jährigen liegenden Normalperson mit den auftretenden Maxima bei 0,1 und ca. 0,25 Hz. Als charakteristisches Ausführungsbeispiel soll nachfolgend die dargestellte Ableitung der Maßzahlen im Sinne der eingeführten Wichtungen präzisiert werden.

Im FFT-Spektrum bilden sich diskrete spektrale Leistungs-Amplituden A(nf$_A$) ab, wobei der Abstand der spektralen Amplituden durch die Abtastfrequenz $f_A$ festgelegt ist. Der einhüllende Verlauf von $A(nf_A)$ sowie die Flächensumme für den jeweiligen definierten Frequenzbereich ist ein Maß für den vegetativen kardiovaskulären Zustand.

1. Schritt: Ableitung von Normalwerten

Die von Normalpersonen abgeleiteten R-R-Tachogramme werden bei einer EKG-Ableitezeit für eine Messung von 120 Sekunden sowie einer Abtastfrequenz von etwa 1000 Hz einer FFT-Analyse unterzogen, so dass sich die Amplituden A $(nf_A)$ abbilden. Da die Ableitezeit konstant ist und auch angenommen werden kann, dass die Anzahl der bei einer Messung jeweils zugrunde liegenden R-R-Intervalle in einem "normalen" Streubereich liegt, werden alle Amplituden $A(nf_A)$ bei den N Normalpersonen "übereinander gelegt" und für jede Frequenz $nf_A$ der zugehörige Mittelwert sowie die Standardabweichung ermittelt. Es erfolgt als Spektrum eine graphische Darstellung dieser Mittelwerte einschließlich Streuung bei jeder Spektrallinie. Danach ist eine Kurvenglättung vorzunehmen, die so erhaltenen Werte sind als Normalwerte A $(nf_A)$ normal im Rechner abzulegen.

Aus Figur 10 läßt sich verallgemeinernd ableiten:

Es tritt im Normalfall in jedem Bereich jeweils 1 Maximum auf:

- im LF-Bereich bei ca. 0,1 Hz
- im HF-Bereich bei ca. 0,25 Hz

Diese jeweiligen Amplitudenmaxima bei den Frequenzen von 0,1 und 0,25 Hz werden als Normwerte der beiden Bereiche LF, HF festgelegt:

$$A_{LF\,max} \text{ sowie } A_{HF\,max}.$$

Jede spektrale Amplitude im jeweiligen Frequenzbereich wird dividiert durch das zugehörige Amplitudenmaximum $A_{LF\,max}$ bzw. $A_{HF\,max}$, so dass sich eine normierte Darstellung ergibt. Somit erhält man sowohl für den LF-, als auch HF-Bereich als jeweiligen Maximalwert den Betrag Eins. Entsprechend wird die Standardabweichung normiert.

Liegt ein beliebiges Spektrum mit den spektralen Amplituden A $(nf_A)_{beliebig}$ vor, so sind diese Werte ebenso zu normieren, also durch die Amplitudenmaxima (Normwerte) $A_{LF\,max}$ bzw. $A_{HF\,max}$ zu dividieren. Somit erhält man ein normiertes und dimensionsloses Spektrum, wo sofort die Abweichungen vom Normalverlauf in beiden Frequenzbereichen qualitativ ersichtlich sind. Übersteigt die spektrale Fläche im Bereich 0,04...0,15 Hz die des Normalverlaufs, so liegt ein anormaler Fall genauso vor, wie bei einem Rückgang der Fläche von 0,15...0,4 Hz im Vergleich zum Normalfall.

2. Schritt: Ableitung von Flächen-Maßzahlen

Das in einem 1. Schritt abgeleitete beliebige und normierte Leistungsspektrum zeigt also mögliche qualitative Unterschiede zum Normalfall. In einem 2. Schritt sollen die quantitativen Maßzahlen hierfür ermittelt werden.

Aus den im 1. Schritt für die jeweiligen Frequenzbereiche 0,04...0,15 und 0,15...0,4 Hz abgeleiteten normierten spektralen Verläufen lassen sich die Flächenintegrale bestimmen, wenn als Integrationsgrenzen 0,04...0,15 Hz sowie 0,15...0,4 Hz gesetzt werden.

Algorithmen:

- Normalfall:

Bestimmung der beiden Flächenintegrale des gemittelten "Normalspektrums":

$$F_{normal\,0,04...0,15\,Hz} = \sum_{0,04...0,15\,Hz} \frac{spektrale\ Amplitude\ A(nf_A)}{A_{LF\,max}},$$

$$F_{normal\,0,15...0,4\,Hz} = \sum_{0,15...0,4\,Hz} \frac{spektrale\ Amplitude\ A(nf_A)}{A_{HF\,max}}.$$

- beliebiger Fall:

Analog gilt für den beliebigen Fall:

$$F_{0,04...0,15 Hz} = \sum_{0,04...0,15 Hz} \frac{spektrale\ Amplitude\ A(nf_A)}{A_{LFmax}} \quad bzw.$$

$$F_{0,15...0,4\ Hz} = \sum_{0,15...0,4\ Hz} \frac{spektrale\ Amplitude\ A(nf_A)}{A_{HFmax}} \quad .$$

- aus den Flächenintegralen abgeleitete spektrale Maßzahlen

Diese Flächenintegrale lassen sich erfindungsgemäß durch Division durch den jeweiligen mittleren Flächen-Normalwert normieren, so dass man "spektrale Maßzahlen M" als quantitative dimensionslose Größen erhält:

$$M_{LF} = \frac{F_{0,04...0,15\ Hz}}{F_{normal\ 0,15...0,4\ Hz}} \quad 100\,[\%]$$

$$M_{HF} = \frac{F_{0,15...0,4\ Hz}}{F_{normal\ 0,04...0,15\ Hz}} \quad 100\,[\%]$$

3. Schritt: Präzisierung der abgeleiteten spektralen Maßzahlen

Erfindungsgemäß wurde dargestellt, wie insbesondere der LF-Frequenzbereich des FFT-Spektrums gewichtet werden kann, um einen dort auftretenden sympathischen im Vergleich zum parasympathischen Aktivitätsanteil zu erkennen. Bezogen auf die zugehörigen Flächenintegrale $F_{0,04...0,15\ Hz}$ sowie $F_{0,15...0,4\ Hz}$ und damit auch auf die dimensionslosen Maßzahlen $M_{LF}$ und $M_{HF}$ sind diese mit dem Quadrat der mittleren Herzfrequenz zu wichten.

Aus der Physiologie ist bekannt, dass als Normalbereich (normaler Sinusrhythmus) der mittleren Herzfrequenz 60 ... 80 min$^{-1}$ zugrunde gelegt wird, wobei von Schmidt und Thews ein mittlerer Normalwert von 72 min$^{-1}$ angegeben wird [Schmidt, R F und G Thews: Physiologie des Menschen. 27. Auflage. Springer Berlin Heidelberg New York 1997]. Als Normierungswert für die durchzuführenden Wichtungen wird die "Mitte" des eingeführten physiologischen Normalbereiches von 70 min $^{-1}$ mit ausreichender Näherung angenommen.

Somit können folgende Präzisierungen der abgeleiteten spektralen Maßzahlen durch entsprechende Wichtungen mit der normierten Herzfrequenz vorgenommen werden, wobei die Herzfrequenz in [min$^{-1}$] zu setzen ist und für die Maßzahlen relative Zahlen gelten:

- 1. Wichtung:

$$M_{LF} \bullet \left[\frac{\overline{f_H}}{70\,\mathrm{min}^{-1}}\right]^2 = M_{LF}^{\ *},$$

$$M_{HF} \bullet \left[\frac{\overline{f_H}}{70\,\mathrm{min}^{-1}}\right]^2 = M_{HF}^{\ *}$$

$$\text{sympatho-vagale Balance } 1 = \frac{M_{LF}^{\:\bullet}}{M_{HF}^{\:\bullet}} = \frac{M_{LF}}{M_{HF}}$$

Eine solche Wichtung verändert die Balance nicht.

- 2. Wichtung:

$$M_{LF} \bullet \left[\frac{\overline{f_H}}{70\,\text{min}^{-1}}\right]^2 = M_{LF}^{\:\bullet} \quad (\textit{Wichtung im Vergleich zum 1. Fall unverändert})$$

$$M_{HF} \bullet \left[\frac{70\,\text{min}^{-1}}{\overline{f_H}}\right]^2 = M_{HF}^{\:\bullet\bullet}$$

$$\textit{sympatho} - \textit{vagale Balance } 2 = \frac{M_{LF}^{\:*}}{M_{HF}^{\:*}}$$

Durch Einsetzen der entsprechenden Maßzahlen folgt

$$\textit{sympatho} - \textit{vagale Balance } 2 = \frac{M_{LF}}{M_{HF}} \bullet \left[\frac{\overline{f_H}}{70\,\text{min}^{-1}}\right]^4 \quad \textit{bzw.}$$

$$\textit{sympatho} - \textit{vagale Balance } 2 = [\textit{sympatho} - \textit{vagale Balance } 1] \bullet \left[\frac{\overline{f_H}}{70\,\text{min}^{-1}}\right]^4$$

Somit erhält man als Verhältnis der gewichteten Balancen 2 und 1

$$\frac{\textit{sympatho} - \textit{vagale Balance } 2}{\textit{sympatho} - \textit{vagale Balance } 1} = \left[\frac{\overline{f_H}}{70\,\text{min}^{-1}}\right]^4 .$$

- 3. Wichtung:

$$M_{LF} \bullet \left[\frac{\overline{f_H}}{70\,\text{min}^{-1}}\right]^2 = M_{LF}^{\:*} \quad (\textit{Wichtung im Vergleich zum 1. Fall unverändert})$$

$$M_{HF}$$

$$sympatho-vagale\ Balance\ 3 = \frac{M_{LF}^{\ *}}{M_{HF}}$$

$$= \frac{M_{LF}}{M_{HF}} \bullet [\frac{\overline{f_H}}{70\,min^{-1}}]^2$$

Als Verhältnis der gewichteten Balancen 3 und 1 folgt unmittelbar

$$\frac{sympatho-vagale\ Balance\ 3}{sympatho-vagale\ Balance\ 1} = [\frac{\overline{f_H}}{70\,min^{-1}}]^2$$

In Figur 11 ist das Verhältnis $V_{Balancen}$ der definierten Balancen in Abhängigkeit der Herzfrequenz dargestellt. Es ist ersichtlich, dass höhere Frequenzen dieses Verhältnis stark erhöhen, während Frequenzen kleiner als der Normalwert von $f_H$ = 70 min$^{-1}$ dieses Verhältnis deutlich reduzieren. Dies stimmt mit dem physiologischen Verhalten völlig überein, da höhere Herzfrequenzen den Sympathikus und damit die Streßkomponente erhöhen, während unter dem Normalwert liegende Frequenzen den Parasympathikus aktivieren und somit der Streß reduziert wird. Als Beispiele seien angeführt (s. Figur 11):

- $\overline{f_H}$ = 50 min$^{-1}$: Eine Wichtung 2 reduziert gegenüber Wichtung 1 das Verhältnis V $_{2;1}$ auf 0,26, die Wichtung 3 auf V $_{3;1}$= 0,51.
- $\overline{f_H}$ = 90 min$^{-1}$: Hier erhöhen sich die Verhältniszahlen auf V $_{2;1}$ = 2,73 bzw. V $_{3;1}$ = 1,65.

Figur 12 a bis e) zeigen als weitere Ausführungsbeispiele bei einer 30jährigen liegenden Versuchsperson (Raucher) die bei jeweils 2 Minuten Ableitezeit erhaltenen Elektrokardiogramme unter Ruhe, reflektorischer Vasokonstriktion, Hyperventilation (vertiefte Brustatmung) unter 6 bzw. 10 min$^{-1}$.

Während unter Ruhe nach Figur 12a) sowohl die Streß- als auch Entspannungs-Maßzahl beträchtlich unter den Normalwerten liegen, die sympatho-vagale Balance 3 = 1,04 jedoch genau im Normalbereich, verändern sich die Verhältnisse in Figur 12b) stark. Durch plötzliches passives Eintauchen der Hand in Eiswasser wird eine sympathische Erregungswelle ausgelöst, die die kardiovaskuläre Mikrozirkulation reduziert, die Herzfrequenz erhöht. Deutlich zeigt sich das bei der Streß-Maßzahl, die sich mit dem typischen Frequenzmaximum bei 0,1 Hz um das 4-fache erhöht, während die Entspannungs-Maßzahl unverändert bleibt. Dadurch erhöht sich die Balance 3 als Ausdruck der ausgelösten sympathischen Erregungswelle auf 4,84. Diese Veränderung ist ein völlig normales kardiovaskuläres Verhalten, eine kardiovaskuläre autonome Neuropathie kann ausgeschlossen werden.

Bei gleicher Person wurden in den Figuren 12 c) und d) die Verhältnisse unter den physiologischen Funktionstests vertiefte Brustatmung (Hyperventilation) mit 6 und 10 min$^{-1}$ im liegenden Zustand abgeleitet. Die unterschiedlichen Frequenzen führen, wie erwartet, zu völlig veränderten Balancen 3. Während sich unter 6 min$^{-1}$ ≡ 0,1 Hz ein deutliches Maximum im LF-Bereich des FFT-Spektrums abbildet, die Herzmikrozirkulation entsprechend reduziert wird, die Streß-Maßzahl $M_{LF}^{\ *}$ sich auf 5,9 und gleichfalls die Balance bei minimaler Entspannungs-Maßzahl auf 15 erhöht wird, ändern sich die Verhältnisse unter der vertieften Atmung von 10 min$^{-1}$ ≡ 0,167 Hz, also im HF-Bereich des FFT-Spektrums gelegen, entgegengesetzt: die Balance geht bedingt durch die erhöhte Entspannungs-Maßzahl von 4,4 bei minimaler Sympathikusaktivität auf den minimalen Wert 0,1.

In Figur 12 e) sind Ausschnitte aus den unter 6 und 10 min$^{-1}$ abgeleiteten Tele-EKG dargestellt. Deutlich ersichtlich sind die unterschiedlichen R-R-Abstände sowohl beim Ein- als auch Ausatmen, jedoch auch bei den kardiovaskulären Kennwerten insgesamt. So bewirkt die tiefe Brustatmung von 6 min$^{-1}$ ≡ 0,1 Hz im Vergleich zu 10 min$^{-1}$ ≡ 0,167 Hz durch die Sympathikusaktivierung eine höhere Herzfrequenz, kürzere Herzperiodendauer und eine größere relative Herzfrequenzvariabilität.

Die Herzperiodendauer-Veränderungen in der Figur 13 wurden überraschend bei Ergometer-Belastungen eines Herzinfarkt-Patienten aus entsprechenden Tele-EKG abgeleitet, die sich jedoch prinzipiell auch bei Rad-Profis unter sehr hoher Belastung zeigten. In den Tachogrammen der Herzperiodendauern ist in diesen Fällen keine Atmungsabhängigkeit der Herzperioden zu erkennen, jedoch verhalten sich dieselben nahezu wie ein elektronischer Multivibrator: es treten periodische Schwingungen mit der Tendenz einer Periodenverdopplung auf. Offensichtlich stellen sich diese Zustände des nichtlinearen Herz-Kreislauf-Systems beim Übergang von Ordnung zu Chaos ein, wie erstmals vom Physiker Feigenbaum im Jahre 1976 entdeckt wurde, als er verschiedene Gleichungen mit Periodenverdopplungsverhalten untersuchte. Feigenbaum erkannte allgemein, dass eine Periodenverdopplung beim Zusammenbruch der Ordnung und beim Übergang ins Chaos eine im Sinne der Chaostheorie typische Erscheinungsform bei nichtlinearen Systemen darstellt, wie sie z. B. auch in chemischen Belusow-Zhabotinsky-Reaktionen gefunden wurde. Wie die periodischen Veränderungen der Herzaktionen in Figur 13 zeigen, sind diese Verhältnisse offenbar auch auf das Herz-Kreislauf-System anwendbar, so dass nach Auftreten einer Herzperiodendauer-Verdopplung als kritischer Zustand ein vollständiges Herzmuskelchaos einsetzen kann.

Wird nun ein R-R-Verlauf entsprechend Figur 13 (re.) einer FFT-Transformation unterzogen, so bilden sich im erweiterten HF-Spektrumsbereich anormal höhere Anteile ab, so dass dieser Bereich von etwa 0,35 ... 0,5 Hz das vorliegende kardiovaskuläre Risiko genauer, jedoch noch nicht ausreichend, beschreibt. Die auftretenden Grenzen sind nicht darstellbar, da ein FFT-Spektrum nur bis 0,5 Hz abgebildet werden kann. Erfindungsgemäß zeigte sich, dass durch eine Frequenzanalyse der abgeleiteten EKG-Gesamtzeitfunktion von 0 bis etwa 2 Hz zu einer Problemlösung führt, wie aus Figur 14 folgt. Tritt eine exakte Herzfrequenzverdopplung bei gleicher Amplitude auf, so liegt der mögliche kritische kardiovaskuläre Zustand mit Umschalten zum Herzmuskelchaos vor. Während zum Zeitpunkt "ohne Belastung" die dominante Frequenz f = 1,234 Hz $\equiv$ 74 min$^{-1}$ beträgt und der mittleren Herzfrequenz entspricht, verändern sich die spektralen Anteile unter Belastung mit etwa 50 W deutlich: Es bildet sich mit f = 0,813 Hz $\equiv$ 49 min$^{-1}$ die mittlere Frequenz der einen mittleren Herzperiodik ab, dabei mit der nahezu gleichen spektralen Amplitude wie die der anderen mittleren Herzfrequenz f = 91 min$^{-1}$. Bei dieser Belastung ist der kritische Zustand offensichtlich noch nicht erreicht. Wird danach die Belastung erneut auf Null gesetzt, dann stellen sich wieder Normalverhältnisse ein.

Aus den Frequenzspektren soll erfindungsgemäß ein (Stabilitäts-)Kriterium $K_\mu$ für das (nichtlineare) Herz-Kreislauf-System abgeleitet werden, um ein einsetzendes Herzmuskelchaos möglichst auszuschließen.

Setzt man anstelle Herzfrequenz $f_H$ die zugehörige Herzperiodendauer $T_H$

$$f_H \, [Hz] \, = \, 1 \, / \, T_H \, [s],$$

so gilt für ein Amplitudenspektrum nach Figur 14

$$f_{H1} \, = \, 1 \, / \, T_{H1}, \, f_{H2} \, = \, 1 \, / \, T_{H2}.$$

Tritt im Grenzfall eine Verdopplung der Herzperiodik nach Figur 13 ein, so gilt dies wegen des reziproken Zusammenhangs für die Frequenz $f_{H2}$, also für $T_{H2}$. Folglich muß frequenzmäßig für Stabilität gelten

$$T_{H2} \, \leq \, 2 \, T_{H1}$$

bzw.

$$f_{H2} \geq \frac{1}{2} \cdot f_{H1}.$$

Im Grenzfall der Herzperiodendauer-Verdopplung gilt frequenzmäßig folglich

$$f_{H2} = \frac{1}{2} f_{H1}.$$

während hierfür die zugehörigen Amplituden $A_{f1}$ und $A_{f2}$ gleichen Betrag aufweisen müssen. Erfindungsgemäß soll daraus näherungsweise als (Stabilitäts-)Kriterium $K_\mu$ für das kardiovaskuläre System, abgeleitet aus der EKG-Zeitfunktion, formuliert werden:

$$K_{Frequenz} = \frac{f_1}{f_2} < 2$$

$$K_{Amplitude} = \frac{A_{f2}}{A_{f1}} < 1$$

Die Darstellung des kardiovaskulären Zustandes durch telemedizinischen Datentransfer auf das Display eines Mobilfunkgerätes bzw. allgemein auf ein peripheres Endgerät als Komponente des telemedizinischen Gesamtsystems "kardialer Komplex-Analyzer" zeigt Figur 15.

Auf 2 tachometerartig gestalteten Oberflächen werden mittlere Herzfrequenz, Herzfrequenzvariabilität, Streß- und Entspannungs-Maßzahlen sowie die gewichtete sympatho-vagale Balance dargestellt, wobei die Meßwerte mit abgeleiteten Normalwerten bzw. -bereichen im liegenden Ruhezustand verglichen werden können. Als Oberflächen-Variante ist dabei der Zeiger feststehend, während die Skalenteile sich entsprechend ihrer Werte veränderlich markieren. Bei der Normalwertermittlung zeigte sich, dass bei den Komponenten Streß, Entspannung sowie Balance von einer logarithmischen Normalverteilung ausgegangen werden kann und somit auch erfindungsgemäß eine entsprechende Gestaltung dieser Skaleneinteilungen möglich wird.

Figur 16 zeigt ein Ausführungsbeispiel, das nicht Teil der vorliegenden Erfindung bildet. Analog zur bekannten Auto- bzw. Flugzeug-Black-Box, wo entsprechende technische Parameter zwecks späterer Auswertung zeitweise gespeichert werden, erfolgt neben der Möglichkeit der direkten telemedizinischen Übertragung und Auswertung in einer Empfangsstation sowie möglicher Rückkopplung durch den Arzt eine Erfassung von Vitaldaten, insbesondere EKG, Blutdruck und Blutsauerstoffsättigung, in einer speziellen medizinischen Daten- und Überwachungsbox im PKW, LKW oder Flugzeug. Die Daten können nach der Erfassung direkt oder mittels Bluethooth-Funktion an die Box zwecks späterer Auswertung gesendet werden. Ersichtlich ist, dass über Satellit eine ständige Fahrzeugortung möglich und somit auch im Notfall eine Kommunikation zwischen entsprechenden Personen im Fahrzeug und der Empfangsstation, speziell auch dem Arzt, herstellbar ist.

In Figur 17 ist der gesamte Kommunikationsprozeß von der EKG-Ableitung und Speicherung sowie des telemedizinischen Datentransfers unter Nutzung der verschiedensten analogen und digitalen Kommunikationswege wie Telefon, Internet, Intranet, Funk, Infrarot, Bluetooth auf zentrale oder dezentrale Server/Datenbanken dargestellt. Weiterführend umfasst der Kommunikationszyklus zudem die Weiterleitung und Visualisierung zu Endnutzern, wie Kliniken, Ärzten anderen Anwendern sowie die Rückkopplung und Reaktion nach erfolgter Diagnostik. Erfindungsgemäß ist eingeschlossen der Abruf der gespeicherten Daten durch PC vom Datenlogger (EKG-Sensor).

Zur Realisierung des vorgestellten Verfahrens ist eine Vorrichtung erforderlich. Dabei zeigt Figur 18 die bekannte allgemeine Prinzipdarstellung eines telemedizinischen EKG-Sensors mit Signalverarbeitung und Speicherung sowie Bedienerschnittstelle und Sendeeinheit.

Eine wesentliche Anforderung für den Einsatz derartiger Systeme ist grundsätzlich eine Vereinfachung der Bedienung, da der Sensor auch von physisch eingeschränkten Patienten selbst, ohne Einsatz von zusätzlichen medizinischem Personal, anzuwenden ist.

Nach Figur 19 wird ein 1-Tasten-Prinzip für das telemedizinische Sensorsystem im obigen Sinne derart realisiert, daß nur eine einzige Taste mit der Power-Logik und gleichzeitig mit einem Microcontroller verbunden ist. Dabei besitzt die Powerlogik eine Selbsthaltefunktion und stellt der Vorrichtung eine Spannung mit dem ersten Tastendruck solange bereit, bis eine Abschaltung erfolgt. Der Initialzustand "AUS" wird beim ersten Tastendruck verlassen, gleichzeitig wird die Spannungsversorgung des Messsystems eingeschaltet. Der erreichte Zustand "Bereitschaft1" aktivierte eine später beschriebene Elektroden-Impedanzmessung oder eine Kapazitätsmessung, die bei sicherem Hautkontakt eines Elektrodenpaares die Aufnahme startet. Wenn innerhalb eines Zeitfensters kein Hautkontakt erkannt wurde, dann wird in den Zustand "AUS" gewechselt. Wird innerhalb dieses Zeitfensters die Taste wiederholt betätigt, wird das Senden der Daten initiiert. Der nach dem Senden erreichte Zustand "Bereitschaft2" ermöglicht eine sofortige Sendewiederholung und informiert den Nutzer akustisch/optisch über eine realisierte Übertragung. Das Ausbleiben weiterer Tastenbetätigungen führt zum Ablauf eines Timers, der das System abschaltet. Mit der vorgestellten Erfindung kann je nach vorgegebener Elektrodenkonfiguration (offen / geschlossen) die Aufnahme gestartet oder abgebrochen werden.

Wie dargestellt wurde besteht ein wesentliches Problem der Telemedizin darin, die zu übertragene Datenmenge auf das notwendige Minimum zu reduzieren. Des weiteren kann davon ausgegangen werden, dass nahezu weltweit prinzipiell flächendeckende Mobilfunknetze zur Verfügung stehen. Die Eigenschaft der asymmetrischen Übertragungskapazitäten in Sende-und Empfangsrichtung begrenzt jedoch eine mobile Datenerfassung, da sie auf den Empfang von Daten optimiert sind und in Senderichtung nur geringere Datenübertragungsraten zur Verfügung stehen.

Bei der vorliegenden telemedizinischen Aufgabenstellung wird postuliert, dass die RR-Abstände bereits auf dem EKG-Sensor aus dem hochaufgelösten Signal erkannt werden. Dadurch ist es möglich, die für das Verfahren notwendige Auflösung von etwa 1 Millisekunde zu erreichen, ohne erheblich mehr redundante Daten übertragen zu müssen. Gleichzeitig wird erreicht, dass die bei üblichen Verfahren mit hohen Kompressionsfaktoren auftretenden Artefakte bedeutungslos werden. Es ist somit möglich, nur noch ein signalgemitteltes EKG, bestehend aus Mittelwert und Standardabweichung, zu übertragen.

Die telemedizinische Aufgabenstellung erfordert die Anwendung von mindestens 2 Elektrodensystemen:

- Festelektroden am Gerät (kein Verbrauchsmaterial notwendig, vorteilhaft bei Anwendung im Wellnessbereich),
- Klebeelektroden (lange Aufnahmezeiten sowie Standardableitungen und unter Belastung bzw. Bewegung möglich, medizinische Anwendung).

Im Sinne der einfachen und sicheren telemedizinischen Anwendung von Festelektroden ist es unerlässlich, den Anpressdruck und somit den Übergangswiderstand Elektrode-Haut während der Messung zu überwachen und gegebenenfalls die Verringerung des Anpressdrucks zu signalisieren.

Bekannte Methoden hierzu sind die Auswertung der DC-Offsets beim Einspeisen einer Gleichspannung mittels Komparator oder die Auswertung einer überlagerten Wechselspannung mit einer vielfach höheren Frequenz als das Nutzsignal (> 20 kHz), die auch über eine zusätzliche Elektrode am Körper eingespeist werden kann. Die erste Methode ist einfach, aber meist nicht zuverlässig genug, die zweite bedingt einen höheren Schaltungsaufwand.

Die Elektrodenerkennung kann wie folgt realisiert werden: Im Gegensatz zu bekannten Lösungen, bei denen ein Prüfsignal eingangsseitig vor dem Instrumentenverstärker bewertet und verarbeitet wird, erfolgt die Analyse des Prüfsignals nach Verstärkung und A/D-Wandlung mittels spezieller Algorithmen der digitalen Signalverarbeitung. Es wird zum eigentlichen Messsignal ein Prüfsignal höherer Frequenz und konstanter Amplitude addiert, welches durch den dem Eingangsspannungsteiler parallel geschalteten Patienten gedämpft wird. Die Amplitude des höherfrequenten Prüfsignals ist also proportional der Impedanz des Elektroden-Haut-Überganges. Das Blockschaltbild zur Amplitudenerkennung ist in Figur 20 dargestellt.

Die Amplitude ist nach der A/D-Wandlung mittels spezieller Algorithmen der digitalen Signalverarbeitung zu berechnen. Hierzu ist es notwendig, das Ausgangssignal des Instrumentenverstärkers jeweils mit Sinus und Cosinus zu multiplizieren. Den beiden harmonischen Funktionen wird als Parameter die Frequenz des Prüfsignals zugewiesen. Zur einfachen Ermittlung des Funktionswertes wird hierbei eine Sinustabelle verwendet, die Anzahl der Elemente dieser Tabelle ergibt sich aus dem Verhältnis von Abtastfrequenz des A/D-Wandlers zu der Frequenz der harmonischen Schwingung. Das Ergebnis beider Multiplikationen wird durch einen Tiefpass gefiltert. Die gesuchte Amplitude ist die Quadratwurzel aus der Summe der Quadrate dieser beiden gefilterten Signale.

Bei einem Verhältnis von Abtast- zu Testfrequenz von vier vereinfacht sich die zur Auswertung der Amplitude des Testsignals notwendige Sinustabelle auf die Werte [0,1,0,-1]. Voraussetzung für diese Vereinfachung ist ein synchroner Takt des A/D-Wandler und D/A-Wandlers. Aus diesem Verhältnis ergibt sich aus einer Abtastfrequenz von 2 kHz eine Prüfsignalfrequenz von 500 Hz.

Bei einer eingespeisten Frequenz von 500 Hz und einer oberen Grenzfrequenz von 1 kHz sowie bei hoher Verstärkung des Differenzverstärkers ist die Amplitude der eingespeisten 500 Hz zu dominant. Dieses Verhalten erfordert eine definierte obere Grenzfrequenz des Differenzverstärkers, die unter 500 Hz liegt und nicht das Nutzsignal dämpft.

Eine Grenzfrequenz des Instrumentenverstärkers wurde durch Optimierung auf 116 Hz festgesetzt, um eine zu große Messamplitude, die dem Nutzsignal überlagert ist, zu verhindern, und damit den Betrieb in der Begrenzung zu verhindern. Der Betrieb in der Begrenzung ist nicht zulässig, da in diesem Fall keine Detektion der Amplitude des 500Hz-Signals den Wert von ~ 0 ergibt und somit einen geringen Übergangswiderstand signalisiert.

Ergebnis dieser Optimierung ist ein vom Instrumentenverstärker verstärktes und anschließend durch einen Tiefpass gedämpftes Prüfsignal. Dieses Prüfsignal verhält sich bei offenen Elektroden wie in Figur 21 ersichtlich. Die Verringerung der Amplitude des Prüfsignals infolge eines geringen Übergangswiderstandes auf ca. 20% geht aus Figur 22 hervor.

**Patentansprüche**

**1.** Verfahren zur Ableitung und Auswertung von Herz-Kreislauf-Informationen aus Herzstromkurven, insbesondere für telemedizinische Anwendungen, wobei aus einem aufgenommenen EKG zur Datenreduktion aus dem hochaufgelösten Signal zunächst über die gesamte Aufnahmezeit ein Mittelwert und eine Standardabweichung als signalgemitteltes EKG bestimmt sowie die R-R-Abstände mittels eines Erkennungsalgorithmus bewertet und nur die erhaltenen Ergebnisse zum Zweck der Datenreduktion übertragen und/oder aufgezeichnet werden, hierfür aus einem FFT-Spektrum der ermittelten Herzperiodendauern Flächenintegrale F für die definierten LF- und HF-Frequenzbereiche von 0,04 bis 0,15 Hz sowie 0,15 bis 0,4 Hz bestimmt und auf die mittlere Flächen-Normalwerte bezogen werden, so dass sich zur Auswertung spektrale Maßzahlen wie folgt ergeben:

$$M_{LF} = \frac{F_{0,04 \ldots 0,15\,Hz}}{mittlerer\ Fl\ddot{a}chen - Normalwert} \cdot 100\,[\%]$$

$$M_{HF} = \frac{F_{0,15 \ldots 0,4\,Hz}}{mittlerer\ Fl\ddot{a}chen - Normalwert} \cdot 100\,[\%] \quad,$$

weiterhin eine Wichtung des Quadrates $s_{TH}^2$ der Standardabweichung, der absoluten Herzfrequenzvariabilität, mit dem Quadrat der mittleren Herzfrequenz $\overline{f_H}$ erfolgt

$$[s_{TH}]^2 \cdot [\overline{f_H}]^2,$$

wodurch der kardiovaskuläre Zustand bezüglich des Einflusses des autonomen Nervensystems mit seinen Komponenten Sympathikus und Parasympathikus bewertbar ist, sowie durch Wichtungen der Maßzahlen $M_{LF}$ und $M_{HF}$ mit dem Quadrat der mittleren Herzfrequenz $\overline{f_H}$ ermittelt wird, wie sich die sympathovagale Balance B verändert und hierdurch die im LF-Frequenzbereich auftretenden sympathischen im Vergleich zu den parasympathischen Aktivitätsanteilen quantifizierbar werden.

2. Verfahren nach Anspruch 1,
   **dadurch gekennzeichnet, dass**
   mit den gewichteten Maßzahlen eine Quantifizierung wie folgt vorgenommen wird:

   • normiertes Maß für sympathische Aktivität, Streß und Belastung:

   $$M_{LF} \bullet \left[\frac{\overline{f_H}}{70\,min^{-1}}\right]^2 = M_{LF}^{\bullet},$$

   • normiertes Maß für parasympathische Aktivität, Entspannung und Erholung:

   $$M_{HF},$$

   • sympatho-vagale Balance:

   $$sympatho - vagale\ Balance = \frac{M_{LF}^{*}}{M_{HF}}$$

   $$= \frac{M_{LF}}{M_{HF}} \bullet \left[\frac{\overline{f_H}}{70\,min^{-1}}\right]^2$$

3. Verfahren nach Anspruch 1,
   **dadurch gekennzeichnet, dass**
   neben der R-R-Bestimmung durch eine Frequenzanalyse der abgeleiteten EKG-Gesamtzeitfunktion im Bereich von 0 bis etwa 2 Hz ermittelt wird, ob zwei dominante Frequenzlinien $f_{1;2}$ in Form einer Frequenzverdopplung bei gleicher Amplitude A $_{f1;2}$ auftreten, um ein Stabilitätsriterium K $_\mu$ für das eigentlich nichtlineare kardiovaskuläre System, abgeleitet aus der EKG-Zeitfunktion, wie folgt festzulegen:

$$K_{Frequenz} = \frac{f_1}{f_2} < 2$$

$$K_{Amplitude} = \frac{A_{f2}}{A_{f1}} < 1$$

## Claims

1. Method for deriving and evaluating cardiovascular information from electrocardiograms, especially for telemedical applications, wherein, for the reduction of data, a mean value and a standard deviation are determined as signal-averaged ECG from the high-resolution signal of a recorded ECG initially over the total recording time and the R-R intervals are evaluated by means of a detection algorithm, and only the received results are transmitted and/or recorded for the purpose of the data reduction, for which purpose area integrals F for the defined LF and HF frequency ranges of 0.04 to 0.15 Hz and 0.15 to 0.4 Hz are determined from an FFT spectrum of the determined heart period durations and are related to the mean area-normal-values, so that spectral indexes for the evaluation are obtained as follows:

$$M_{LF} = \frac{F_{0,04\ ...\ 0,15\ Hz}}{mean\text{-}area\text{-}normal\text{-}value}\ 100\,[\%]$$

$$M_{HF} = \frac{F_{0,15\ ...\ 0,4\ Hz}}{mean\text{-}area\text{-}normal\text{-}value}\ 100\,[\%]$$

wherein further a weighting of the square $s_{TH}^2$ of the standard deviation, the absolute heart rate variability, with the square of the mean heart rate $\overline{f_H}$ is performed

$$[s_{TH}]^2 \cdot [\overline{f_H}]^2,$$

so that the cardiovascular state can be evaluated with respect to the influence of the autonomic nervous system with its components sympathicus and parasympathicus, and wherein it is determined by weightings of the indexes $M_{LF}$ and $M_{HF}$ with the square of the mean heart rate $\overline{f_H}$ how the sympatho-vagal balance B varies, so that the sympathetic activity components occurring in the LF frequency range become quantifiable as compared to the parasympathetic activity components.

2. Method according to claim 1,
**characterized in that**
a quantification is performed with the weighted indexes as follows:

• normalized dimension for sympathetic activity, stress and strain:

$$M_{LF} \bullet [\frac{\overline{f_H}}{70\,\mathrm{min}^{-1}}]^2 = M_{LF}^{\ *},$$

22

• normalized dimension for parasympathetic activity, relaxation and recovery:

$$M_{HF},$$

• sympatho-vagal balance:

$$\text{sympatho-vagal balance} = \frac{M_{LF}^{\ *}}{M_{HF}}$$

$$= \frac{M_{LF}}{M_{HF}} \bullet \left[\frac{\overline{f_H}}{70\,\text{min}^{-1}}\right]^2$$

**3.** Method according to claim 1,
**characterized in that**
beside the R-R determination it is determined by a frequency analysis of the derived ECG total time function in the range of 0 to about 2 Hz whether two dominant frequency lines $f_{1;2}$ in the form of a frequency doubling occur at a same amplitude $A_{f1;2}$, so as to define a stability criterion $K_\mu$ for the actually non-linear cardiovascular system, derived from the ECG time function, as follows:

$$K_{frequency} = \frac{f_1}{f_2} < 2$$

$$K_{amplitude} = \frac{A_{f2}}{A_{f1}} < 1$$

**Revendications**

**1.** Procédé pour la déduction est l'évaluation d'informations cardio-vasculaires à partir de courbes du courant cardiaque, en particulier pour des applications télémédicales, dans lequel on détermine à partir d'un électrocardiogramme enregistré, en vue d'effectuer une réduction de données depuis le signal à haute résolution, tout d'abord sur la totalité du temps d'enregistrement une valeur moyenne et un écart standard, à titre d'électrocardiogramme à signal moyen, et on valorise les écarts R-R au moyen d'un algorithme de reconnaissance et on transmet uniquement les résultats obtenus dans le but d'effectuer une réduction des données et/ou on les enregistre, en déterminant dans ce but, à partir d'un spectre FFT des durées déterminées des périodes cardiaques, des intégrales de surface F pour les plages de fréquences définies LF et HF de 0,04 à 0,15 Hz ainsi que de 0,15 à 0,4 Hz, et on les rapporte aux valeurs normales moyennes vis-à-vis de la surface, de sorte que l'on obtient des nombres spectraux en vue de l'évaluation, comme suit :

$M_{LF}$ = (F $_{0,04...\,0,15\,Hz}$)/(surfaces moyennes - valeur normalisée) 100 [%] $M_{HF}$ = (F $_{0,15...0,4\,Hz}$)/(surfaces moyennes - valeur normalisée) 100 [%] on effectue ensuite une pondération du carré $S_{TH}^2$ de l'écart standard, de la variabilité de fréquence cardiaque absolue, avec le carré de la fréquence cardiaque moyenne f$\square_H\square$
[$S_{TH}$]$^2$ · [f$\square_H\square$]$^2$
grâce à quoi l'état cardio-vasculaire peut être évalué vis-à-vis de l'influence du système nerveux autonome avec ses composantes sympathique et parasympathique, ainsi que par pondération des nombres $M_{LF}$ et $M_{HF}$ avec le carré de la fréquence cardiaque moyenne f$\square_H\square$, on détermine de quelle manière l'équilibre sympathico-vagal B varie et grâce à cela il est possible de quantifier les parts d'activité sympathique qui se produisent dans la plage de fréquence LF par comparaison aux parts d'activité parasympathique.

**2.** Procédé selon la revendication 1,
**caractérisé en ce qu'**avec les nombres pondérés on procède à une quantification, de la manière suivante :

- mesure normalisée pour l'activité sympathique, le stress et les charges :

$$M \cdot [(f\square_{H\square})/(70 \text{ min}^{-1})]^2 = M_{LF}*$$

- mesure normalisée pour l'activité parasympathique, la détente et le repos :

$M_{HF}$

- équilibre sympathique-vagal :

$$\text{équilibre sympathique-vagal} = M_{LF} */M_{HF} = (M_{LF})/(M_{HF}) \cdot [(f\square_{H\square})/(70 \text{ min}^{-1})]^2.$$

**3.** Procédé selon la revendication 1,
**caractérisé en ce que**, outre la détermination R-R, on détermine par une analyse de fréquence de la fonction temporelle globale d'électrocardiogramme dérivée, dans la plage de 0 à environ 2 Hz, s'il se produit deux lignes de fréquence dominantes f $_{1;2}$ sous la forme d'un doublage de fréquence pour la même amplitude A $_{f1;2}$, afin de fixer un critère de stabilité K$\mu$ pour le système cardio-vasculaire en fait non linéaire, dérivé à partir de la fonction temporelle de l'électrocardiogramme, de la manière suivante :

$$K_{\text{fréquence}} = (f1)/(f2) < 2$$

$$K_{\text{amplitude}} = (A_{f2})/(A_{f1}) < 1.$$

EKG-Sensor

Festnetz

Mobil

Satelit

Empfangszentrale
Visualisierung

AUSWERTUNG
Arzt

**Figur 1**

R

P

T

(U)

Q

S

ST-
Strecke

P-Welle
< 0.1 s

T-Welle

0.15 0.1 0.05 0 0.05 0.1 0.15 0.2 0.25 0.3 0.35 0.4 Zeit [s]

| PO-Intervall < 0.2 s | QRS-Gruppe < 0.1 s | |
|---|---|---|

QT-Intervall:
- abhängig von Herzfrequenz
- bei 70 / min: QT = 0.32 ... 0.39 s

**Figur 2**

R1   R2   R3   R4   R5

1   2   3   4

RR-Abstand = Herzperiodendauer

**Figur 3**

25

Figur 4

**Figur 5**

vegetative Komponenten des kardiovaskulären Systems

**Figur 6**

**Figur 7**

**Figur 8**

EP 2 059 163 B1

## spektrale Maßzahlen

**Normalperson** (I: 50, II: 130)
**Koronarpatient** (I: 90, II: 30)

I: Aktivität
Sympathikus,
Stress,
Belastung,
Anspannung

II: vagale
Aktivität,
Entspannung,
Erholung

**Figur 9**

LF-Maximum bei ca. 0,1 Hz

HF-Maximum bei ca. 0,25 Hz

FFT

0   0.1   0.2   0.3   0.4   0.5

LF-Bereich   HF-Bereich

**Frequenz [Hz]**

**Figur 10**

29

Figur 11

**Figur 12 a), b)**

**Figur 12 c), d)**

Figur 12 e)

## ohne Belastung

mittlere Herzperiodendauer = 805 ms
mittlere Herzfrequenz = 75 [min$^{-1}$]
Herzfrequenzvariabilität UHF = 2.5 %

## Belastung mit ca. 50W

mittlere Herzperiodendauer = 656 ms
mittlere Herzfrequenz = 91 [min$^{-1}$]
Herzfrequenzvariabilität UHF = 9.2 %

Figur 13

34

**ohne Belastung**

100% der Maximalamplitude bei f = 1.234 Hz ≙ 74 min⁻¹

aus der EKG-Zeitfunktion abgeleitetes Frequenzspektrum mit der mittleren Herzfrequenz f = 74min⁻¹

**Belastung mit ca. 50W**

100% der Maximalamplitude bei f = 1.516 Hz ≙ 91 min⁻¹

Frequenzspektrum, woraus als mittlere Herzfrequenz $f_1$ = 91 min⁻¹ folgt, als mittlere Herzfrequenz $f_2$ = 49 min⁻¹

Figur 14

**Figur 15**

Satellit
GPS-Fahrzeugortung

medizinische Daten-
und Überwachungsbox

Übertragung per Bluetooth
zur Daten- und Überwachungsbox

Sende- und Empfangsanlage
GSM-Netz / Internet

Empfangsstation
Auswertung Arzt

**Figur 16**

INTERNET

AUTOMATISCHER DATENVERSAND
NACH ABRUFBESTÄTIGUNG

VERSAND SERVER

PAKET A
PAKET B

PC des Arztes / Nutzers

DATEN DOWNLOAD
DURCH PASSWORT

WEB SERVER

ÜBERTRAGUNG
PER IRDA DIREKT ZUR
PC-SCHNITTSTELLE

Dienstleistungszentrum

DATENBANK

ÜBERTRAGUNG
PER IRDA
MOBILFUNKTELEFON

DATENAUFZEICHNUNG

ÜBERTRAGUNG
PER AKUSTIK
ÜBER FESTNETZ

EMPFANG SERVER

**Figur 17**

**Figur 18**

**Figur 19**

**Figur 20**

**Figur 21**

**Figur 22**

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- DE 19749768 A1
- DE 9803225 W
- US 9909336 W
- DE 19830316 A1
- WO 9904687 A
- DE 19902253 A1
- US 9903666 W
- US 9816693 W
- EP 0760225 A1
- EP 0800787 A1
- EP 0855167 A1
- EP 0864294 A2
- GB 9800742 W
- DE 19523199 A1
- DE 19510382 A1
- US 9602826 W
- BE 9500123 W
- EP 0657136 A1
- DE 19954856 A1
- DE 20008602 U1

- AU 0000656 W
- US 9918296 W
- EP 0746229 T1
- IL 0000506 W
- EP 1110502 A2
- DE 10065578 A1
- DE 10247435 A1
- EP 1157659 A1
- DE 10245143 A1
- DE 10233071 A1
- WO 0224068 A1
- DE 20118850 U1
- DE 20309617 U1
- DE 20306009 U1
- DE 29924092 U1
- EP 0739181 B1
- EP 0906058 B1
- DE 69617564 T2
- EP 1459681 A
- DE 102006002045 A

### In der Beschreibung aufgeführte Nicht-Patentliteratur

- **Baumert, J-H ; A W Frey ; M Adt.** Analyse der Herzfrequenzvariabilität. Grundlagen, Methodik und mögliche Anwendung in der Anästhesie. *Anaestesist,* 1995, vol. 44, 677-686

- *European Heart Journal,* 1996, vol. 17, 354-381
- Deutsche Zeitschrift für Sportmedizin. 2005
- **Schmidt, R F ; G Thews.** Physiologie des Menschen. Springer, 1997